# EUROPÄISCHE PATENTANMELDUNG

(11) **EP 2 080 756 A2**
(43) Veröffentlichungstag der Anmeldung: **22.07.2009**
(21) Anmeldenummer: 09002883.8
(22) Anmeldetag: 02.02.2006
(51) Int. Cl.: C07D 233/54, C07D 403/10, C07D 257/04, C07C 251/08

(54) **Verfahren zur Herstellung von Losartan**

(30) Priorität: 03.02.2005 DE 102005005047
(62) Teilanmeldung aus: 06705893.3
(71) Anmelder: Ratiopharm GmbH, 87079 Ulm (DE)
(72) Erfinder: Wang, Yaping, Lanzhou City, Gansu Province 730020 (CN); Li, Yonggang, Pudong district, Shangai 201203 (CN); Li, Yulin, Lanzhou City, Gansu Province 730000 (CN); Zheng, Guojun, Zhejiang Province 31620 (CN); Li, Xi, Xi Guan Group 7, Zheng Ning County Gansu Province 745300 (CN)
(74) Vertreter: Breuer, Markus

(57) **Zusammenfassung**

Die vorliegende Erfindung betrifft ein neues Verfahren zur Herstellung von Losartan, ein Imidazolderivat mit dem chemischen Namen 2-n-Butyl-4-chloro-5-hydroxymethyl-1-{[2'-(1H-tetrazol-5-yl)biphenyl-4-]methyl}imidazol sowie pharmakologisch wirksamer Salze hiervon. Weiterhin betrifft die Erfindung neue Zwischenprodukte, die zur Herstellung von Losartan geeignet sind sowie neue Verfahren zur Herstellung von Zwischenverbindungen, die zur Herstellung von Losartan geeignet sind.

Ein Aspekt der Erfindung ist ein Verfahren zur Herstellung einer Verbindung der allgemeinen Formel I 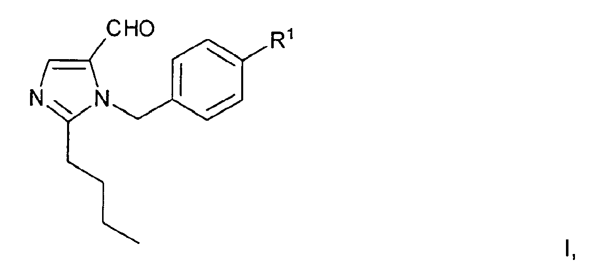 die bei einer erfindungsgemäßen Darstellung von Losartan als Zwischenstufe entstehen kann.

## Beschreibung

Die vorliegende Erfindung betrifft ein neues Verfahren zur Herstellung von Losartan, ein Imidazolderivat mit dem chemischen Namen 2-n-Butyl-4-chloro-5-hydroxymethyl-1-{[2'-(1H-tetrazol-5-yl)biphenyl-4-]methyl}imidazol sowie pharmakologisch wirksamer Salze hiervon. Weiterhin betrifft die Erfindung neue Zwischenprodukte, die zur Herstellung von Losartan geeignet sind sowie neue Verfahren zur Herstellung von Zwischenverbindungen, die zur Herstellung von Losartan geeignet sind.

Losartan und effiziente und wirtschaftliche Wege zu seiner Herstellung sind von großem Interesse, denn Losartan hat sich als potenter Wirkstoff zur Bekämpfung von Bluthochdruck bei Säugetieren einschließlich Menschen und hieraus entstehender Folgeerkrankungen erwiesen.

Losartan und seine Herstellung wurde erstmals in EP-A-253310 beschrieben. Die Synthese umfasste als wesentlichen Schritt eine N-Alkylierung, die Reaktion eines Imidazols mit z.B. einem Bromomethyl-biphenyl-derivat (EP 253310 B1, S. 213, Anspruch 6).

In der EP-A-291969 sind Trityl-geschützte Tetrazolderivate beschrieben, die zur Herstellung von Losartan geeignet sind.

Die WO 03/093262 betrifft die Herstellung von Losartan ausgehend von Tritylgeschützten Tetrazolderivaten durch Abspaltung der Schutzgruppe.

Die Herstellung von Losartan-Kalium, der üblichen Handelsform, aus Losartan ist vielfältig beschrieben (vgl. z.B. EP 324377 A, Seite 191, example 316, part D und WO 95/17396, Seite 18, example 4 und Seite 24, example 9, step C).

Die oben genannten Synthesewege erscheinen jedoch noch verbesserungsbedürftig, um Losartan im industriellen Maßstab herzustellen, denn die Gesamtausbeute lässt noch zu Wünschen übrig.

Allen Syntheserouten ist gemeinsam, dass zunächst ein 1-H-Imidazolderivat hergestellt wird, das anschließend an der 1-Position alkyliert wird. Bei dieser Reaktion besteht jedoch die Möglichkeit, dass zwei Isomere gebildet werden, je nach dem welches der beiden Stickstoffatome alkyliert wird.

Aus J. Org. Chem. 1997, 62(24), 8449-8454 (s. Tabelle 1) ist der gezielte Aufbau eines in der 1-Position alkylierten Imidazolderivates aus einem *N*-monosubstituierten Amidin bekannt. Die Herstellung von geeigneten Vorstufen für die Losartan-Synthese wurde jedoch nicht berichtet.

Aufgabe war es deshalb, neue Synthesewege und Zwischenprodukte für die Herstellung von Losartan und seiner pharmakologisch wirksamen Salze bereitzustellen. Insbesondere war es Aufgabe der Erfindung, neue Synthesewege und Zwischenprodukte für die Herstellung von Losartan und seiner pharmakologisch wirksamen Salze bereitzustellen, mit denen Losartan in einer hohen Gesamtausbeute herstellbar ist.

Ferner war es Aufgabe der Erfindung, neue Synthesewege und Zwischenprodukte für die Herstellung von Losartan und seiner pharmakologisch wirksamen Salze bereitzustellen, die mit geringem apparativen Aufwand auch im technischen Maßstab hergestellt werden können. Weiterhin sollten weitgehend industriell gut zugängliche Einsatzstoffe verwendet und der Einsatz von toxischen oder kennzeichnungspflichtigen Stoffen vermieden werden.

Demgemäß wurden die eingangs beschriebenen Gegenstände gefunden.

Ein zentraler Aspekt der Erfindung ist die Herstellung einer Verbindung der allgemeinen Formel I 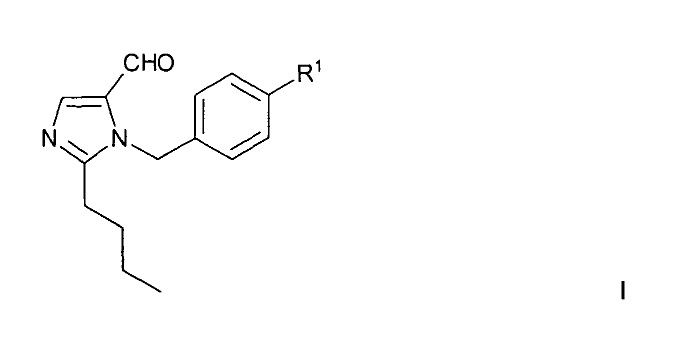 in der R¹ ein Rest R^{1a} oder Rest R^{1b} bedeutet.

Bei R^{1a} handelt es sich um einen Rest der allgemeinen Formel II, 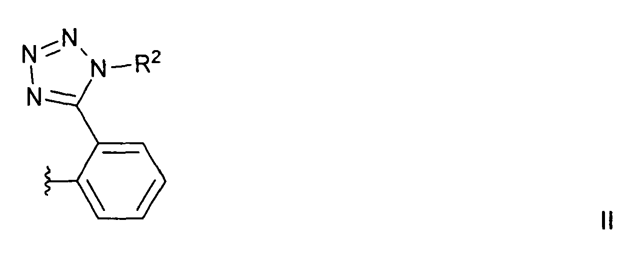 in der R² eine Tetrazol-Schutzgruppe bedeutet.

In der allgemeinen Formel II symbolisiert die "Schlangenlinie" den Verknüpfungspunkt, beispielsweise zur Verbindung gemäß der allgemeinen Formel I.

Geeignete Tetrazol-Schutzgruppen in dem Rest der oben angegebenen allgemeinen Formel II sind aus der EP-A-291969 und WO 03/093262 bekannt (vergl. dort den Triarylmethylsubstituenten in der Verbindung der allgemeinen Formel (II)). Geeignete Tetrazol-Schutzgruppen sind insbesondere Triphenylmethyl oder *tert*.-Butyl.

Bei dem Rest R^{1b} der allgemeinen Formel I handelt es sich um einen Rest, der in der Lage ist, die Phenylengruppe der Verbindung der allgemeinen Formel I mittels einer C-C-Kupplung an eine weitere Arylgruppe zu binden.

Insbesondere handelt es sich bei dem Rest R^{1b} der allgemeinen Formel I um einen Rest, der in der Lage ist, die Phenylengruppe der Verbindung der allgemeinen Formel I durch Reaktion mit einem hierzu komplementären Rest R³, der Bestandteil einer eine andere Phenyleneinheit enthaltende Verbindung der allgemeinen Formel III ist,

R³-R⁴ III

in der R⁴ ein Rest der allgemeinen Formel II bedeutet,
unter Bildung einer C-C-Bindung zwischen der Phenylengruppe der Verbindung der allgemeinen Formel I und der Phenylengruppe der Verbindung der allgemeinen Formel III zu kuppeln. Die C-C-Kupplung erfolgt hierbei üblicherweise unter Abspaltung der Reste R^{1b} und R³.

Die Verbindung der allgemeinen Formel I wird hergestellt, indem man eine Verbindung der allgemeinen Formel IV 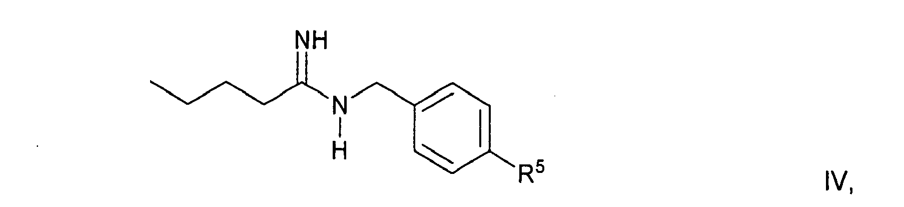 in der R⁵
- für den Fall, dass R¹ in Formel I ein Rest R^{1a} ist, ein Rest der allgemeinen Formel II bedeutet, und
- für den Fall, dass R¹ in Formel 1 ein Rest R^{1b} ist, die gleiche Bedeutung wie Rest R^{1b} in Formel I hat
mit einer Verbindung der der allgemeinen Formel V, 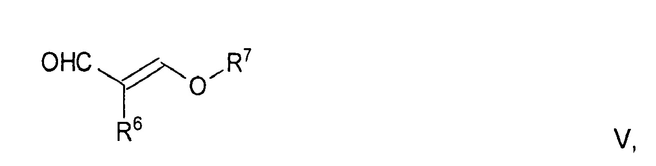 in der R⁶ ein Halogen aus der Gruppe Cl, Br, I, bevorzugt Br, bedeutet und R⁷ eine verzweigte oder unverzweigte C₁ - C₆-Alkylgruppe, bevorzugt eine lsopropylgruppe, bedeutet, umsetzt.

Die vorstehend beschriebene Reaktion (Umsetzung von Verbindung der allgemeinen Formel IV mit Verbindung der allgemeinen Formel V) wird bevorzugt in Gegenwart einer Bronstedt-Base, insbesondere einer schwachen Bronstedt-Base durchgeführt. Geeignete Bronstedt-Basen sind Alkalicarbonate oder Alkalihydrogencarbonate, wie beispielsweise Natriumcarbonat, Kaliumcarbonat oder Natriumhydrogencarbonat. Bevorzugt ist Kaliumcarbonat.

Bevorzugt wird die Reaktion in einem 2-phasigen System, bei dem eine Phase aus einer wässrigen Lösung gebildet ist und die andere Phase aus einer Lösung gebildet wird, die ein mit Wasser nicht unbegrenzt mischbares organisches Lösungsmittel umfasst. Beispiele für geeignete Lösungsmittel sind Toluol, Methylenchlorid, Chloroform oder Gemische daraus.

Die Umsetzung einer Verbindung der allgemeinen Formel IV mit einer Verbindung der allgemeinen Formel Verfolgt üblicherweise in einem molaren Mengenverhältnis von 0,5 bis 2 zu 1, bezogen auf die molaren Mengen an Verbindung der allgemeinen Formel IV zu Verbindung der allgemeinen Formel V.

Die Reaktionsdauer beträgt im Allgemeinen 0,1 bis 20 Stunden, bevorzugt 5 bis 15 Stunden.

Nachstehend wird nun der Rest R^{1b}, der sowohl in Verbindungen der allgemeinen Formel I als auch in Verbindungen der allgemeinen Formel IV (als Rest R⁵) enthalten sein kann, näher erläutert:

Bevorzugt ist der Rest R^{1b} der Verbindung der allgemeinen Formel I oder Rest R⁵ in der Verbindung der allgemeinen Formel IV ein Rest, der in der Lage ist, mit dem Rest R³ unter Bildung einer C-C-Kupplung zur reagieren. Insbesondere ist der Rest R^{1b} der Verbindung der allgemeinen Formel I oder Rest R⁵ in der Verbindung der allgemeinen Formel IV ein Rest, der in der Lage ist, mit dem Rest R³ in einer Suzuki-, Stille- oder Grignard-Reaktion zu reagieren.

Die Begriffe "Suzuki-Reaktion", "Stille-Reaktion" oder "Grignard-Reaktion" sind dem Fachmann allgemein bekannt und beispielsweise in Chem. Rev. 2002, 102(5), 1359-1470 beschrieben.

Besonders bevorzugt hat der Rest R^{1b} in der Verbindung der allgemeinen Formel I oder Rest R⁵ in der Verbindung der allgemeinen Formel IV die folgende Bedeutung:
- Halogen, insbesondere Brom,
- ein Rest der allgemeinen Formel VI 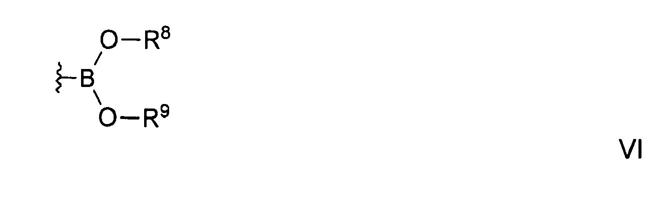 in der R⁸ und R⁹ Wasserstoff, eine C₁- bis C₆-Alkylgruppe oder gemeinsam eine C₁- bis C₆-Alkandiylgruppe bedeutet,
- ein Trialkylzinnrest, wobei bevorzugt "Alkyl" für einen C₁ bis C₁₂, insbesondere C₁ bis C₆ Alkylrest steht, oder
- wenn in dem Verfahren eine Verbindung der allgemeinen Formel I mit dem Rest R^{1b} verwendet wird, ein Magnesium(II)halogenidrest,
und wobei,
wenn R^{1b} oder R⁵ für ein Halogen steht, R³ für einen Rest der allgemeinen Formel VI, einen Trialkylzinnrest oder, wenn in dem Verfahren eine Verbindung der allgemeinen Formel I mit dem Rest R^{1b} verwendet wird, einen Magnesium(II)-halogenidrest steht und umgekehrt.

In dem Rest der allgemeinen Formel VI bedeutet bevorzugt der Rest R⁸ und R⁹ gemeinsam 2,3-Dimethylbutan-2,3-diyl.

Besonders bevorzugt ist weiterhin der Rest R³ aus der allgemeinen Formel III ausgewählt aus der Gruppe umfassend die folgenden Reste:
- Halogen, bevorzugt Brom
- ein Rest der allgemeinen Formel VI
- in Trialkylzinnrest, wobei bevorzugt "Alkyl" für einen C₁ bis C₁₂, insbesondere C₁ bis C₆ Alkylrest steht, oder
- ein Magnesium(II)halogenidrest, bevorzugt ein Magnesium(II)bromidrest, jedoch mit folgender Maßgabe, dass
   die Reste R^{1b} und der Rest R⁵ einerseits und der Rest R³ andererseits so ausgewählt werden, dass Rest R^{1b} und Rest R⁵ einerseits und Rest R³ andererseits komplementäre Reste sind, d.h. sie ein komplementäre Paare bilden, welche miteinander in gewünschter Weise in einer Kupplungsreaktion reagieren können.

Somit sind die Reste R^{1b} und R³ oder R⁵ und R³ so zu wählen, dass sie komplementäre Paare bilden. Bevorzugte komplementäre Paare sind:
a) Halogen und ein Rest der allgemeinen Formel VI; denn sie können beispielsweise in einer Suzuki-Reaktion miteinander reagieren,
b) Halogen und ein Trialkylzinnrest, wobei bevorzugt "Alkyl" für einen C₁ bis C₁₂, insbesondere C₁ bis C₆ Alkylrest steht; denn sie können beispielsweise in einer Stille-Reaktion miteinander reagieren, und
c) Halogen und ein Magnesium(II)halogenidrest; denn sie können in einer Grignard-Reaktion miteinander reagieren.

Halogen steht bevorzugt für Brom und der Magnesium(II)halogenidrest steht bevorzugt für einen Magnesium(II)bromidrest.

Bei den Suzuki-, Stille- oder Grignardreaktionen, die bei den erfindungsgemäßen Herstellungsverfahren zum Einsatz kommen, werden mit Vorteil ein oder mehrere Katalysatoren eingesetzt. Die Katalysatoren können ein oder mehrere Übergangsmetalle umfassen, insbesondere Mangan, Chrom, Eisen, Kobalt, Nickel oder Palladium. Vorzugsweise eingesetzte Verbindungen sind ausgewählt aus MnCl₂, CrCl₃, FeCl₂, Fe(acac)₃, FeCl₃, Fe(salen)Cl, NiCl₂(PPh₃)₂, CoCl₂(dppe), CoCl₂(dpph), Co(acac)₂, COCl₂(dppb), PdCl₂(PPh₃)₂ oder Pd(PPh₃)₄.

Mit Vorteil werden die Katalysatoren zusammen mit einem Aktivator und/oder Stabilisator verwendet. Der Aktivator überführt die Metallatome der Katalysatoren in die Oxidationsstufe null und der Stabilisator stabilisiert die Metallatome der Katalysatoren in der Oxidationsstufe null. Beispiele für derartige Aktivatoren sind Zink (bevorzugt in Form von Zinkpulver), Natriumborhydrid, Lithiumaluminiumhydrid oder organische Verbindungen von Aluminium, Magnesium oder Lithium (bevorzugt Butyllithium oder DIBAH). Beispiele für derartige Stabilisatoren sind Lewis-Basen, bevorzugt Phosphane, besonders bevorzugt Triarylphosphane und Trialkylphosphane, insbesondere Triphenylphosphan.

Bei der Suzuki-Reaktion wird besonders vorteilhaft ein Palladium-Katalysator wie Pd(PPh₃)₄ als Katalysator eingesetzt. Sie erfolgt bevorzugt in Gegenwart einer schwachen Bronstedt-Base wie einem Alkalicarbonat. Mit Vorteil wird die Reaktion in einem 2-phasigen System, bei dem eine Phase aus einer wässrigen Lösung gebildet ist und die andere Phase aus einer Lösung gebildet wird, die ein mit Wasser nicht unbegrenzt mischbares organisches Lösungsmittel wie Benzol, Toluol, Xylol, Methylenchlorid oder Chloroform umfasst, durchgeführt.

Bei der Stille-Reaktion wird besonders vorteilhaft ein Palladium-Katalysator wie Pd(PPh₃)₄ oder PdCl₂(PPh₃)₂ als Katalysator eingesetzt. Die Reaktion erfolgt bevorzugt bei erhöhter Temperatur, vorzugsweise bei Temperaturen zwischen 50 °C und dem Siedepunkt des Lösungsmittels. Mit Vorteil wird die Reaktion in Gegenwart eines Co-Katalysators wie Cul (Kupferiodid) oder CuO (Kupferoxid) durchgeführt. Die Reaktion wird vorzugsweise in inerten Lösungsmitteln wie beispielsweise Toluol, Xylol, Dimethoxyethan, Dimethylformamid, Tetrahydrofuran, oder Dioxan durchgeführt.

Bei der Grignard-Reaktion wird besonders vorteilhaft ein Palladium-Katalysator wie Pd(PPh₃)₄, PdCl₂(PPh₃)₂ oder NiCl₂(PPh₃)₂ als Katalysator eingesetzt. Die Reaktion wird vorzugsweise in polaren, aprotischen Lösungsmitteln wie beispielsweise Tetrahydrofuran, Diethylether oder Dioxan durchgeführt.

Verbindungen der allgemeinen Formeln I werden im Allgemeinen wie vorstehend beschrieben durch Umsetzung von Verbindungen der allgemeinen Formel (IV) mit Verbindungen der allgemeinen Formel (V) hergestellt. Je nach Art der Reste R¹ und R⁵ sind unterschiedliche Syntheserouten bevorzugt.

Im Rahmen dieser Erfindung werden drei bevorzugte Syntheserouten näher beschrieben, die als Syntheseroute A, Syntheseroute B und Syntheseroute C bezeichnet werden.

Syntheseroute A beschreibt den Fall, dass R⁵ in Verbindungen der allgemeinen Formel IV ein Rest der allgemeinen Formel II bedeutet. (Das heißt, der Rest mit R⁵ in Formel IV entspricht den Rest R^{1a} in Formel I.)

Im Allgemeinen erfolgt die Herstellung der Verbindung der allgemeinen Formel IV, in der R⁵ ein Rest der allgemeinen Formel II bedeutet, indem man eine Verbindung der allgemeinen Formel VII 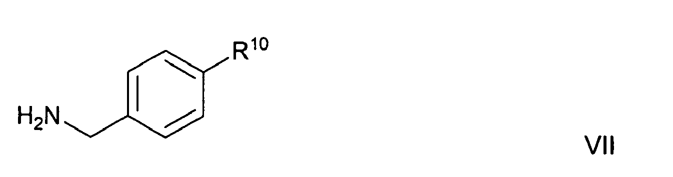 in der R¹⁰ ein Rest der Formel II bedeutet,
mit einer Verbindung der allgemeinen Formel VIII 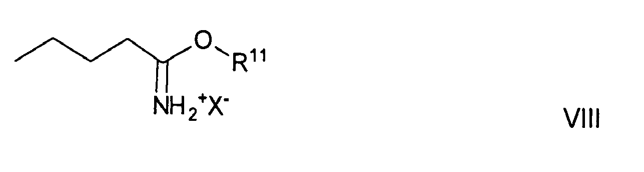 in der R¹¹ ein C₁- bis C₁₂-Alkylrest und X- das Anion einer Mineralsäure bedeutet,
bevorzugt in Gegenwart einer Bronstedt-Base umsetzt.

Die Reaktion wird üblicherweise in einem geeigneten inerten Lösungsmittel, z.B. einem aliphatischen Alkohol wie Ethanol durchgeführt. Als Bronstedt-Base eignet sich z.B. ein tertiäres aliphatisches Amin wie Triethylamin zur Neutralisierung.

Die Umsetzung erfolgt üblicherweise in einem molaren Mengenverhältnis von 0,3 bis 3 zu 1, bezogen auf die molare Menge an Verbindung der allgemeinen Formel VII im Verhältnis zu Verbindung der allgemeinen Formel VIII. Die Reaktionsdauer beträgt im Allgemeinen 0,1 bis 20 Stunden, bevorzugt 5 bis 15 Stunden.

Besonders vorteilhaft erfolgt die Herstellung der Verbindung der allgemeinen Formel VII, indem man:
I. eine Verbindung der allgemeinen Formel IX 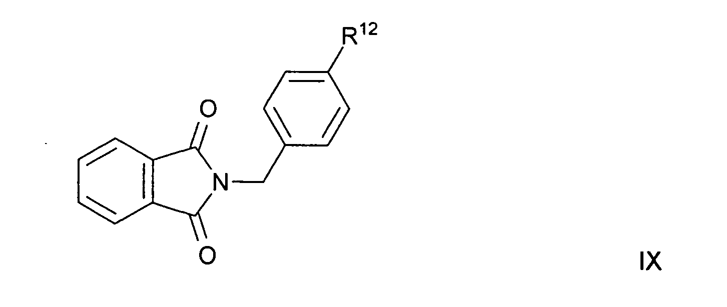 bereitstellt, in der R¹² ein Rest der allgemeinen Formel II ist und
II. aus der Verbindung der allgemeinen Formel IX unter Bedingungen, wie sie für eine Gabriel-Reaktion üblich sind, die Verbindung der allgemeinen Formel VII herstellt. Hierzu wird die Verbindung der allgemeinen Formel IX z.B. mit Hydrazinhydrat in alkoholischer Lösung umgesetzt.

Die Herstellung einer Verbindung der allgemeinen Formel IX ist z.B. aus Bioorganic & Medicinal Chemistry Letters 1993, 3(4), 757-760 (dort als Verbindung 20 bezeichnet) bekannt.

Die Herstellung einer Verbindung der allgemeinen Formel VIII ist z.B. aus J. Org. Chem. 1999, 64(22), 8084-8089 bekannt.

Syntheseroute B beschreibt den Fall, dass R⁵ in Verbindungen der allgemeinen Formel IV ein Halogenatom, insbesondere Brom, bedeutet. (Das heißt, der Rest R⁵ in Formel IV entspricht den Rest R^{1b} in Formel I.)
Zur Herstellung einer Verbindung der allgemeinen Formel IV, in der R⁵ Halogen bedeutet, setzt man bevorzugt ein in *para*-Stellung mit einem Halogenatom substituiertes Benzylaminderivat (d.h. eine Verbindung, die der allgemeinen Formel VII entspricht) mit einer Verbindung der allgemeinen Formel VIII bevorzugt in Gegenwart einer Bronstedt-Base um. Für die Bedingungen dieser Umsetzung gilt das Gleiche wie für die Herstellung der Verbindung der allgemeinen Formel IV, in der R⁵ ein Rest der allgemeinen Formel II bedeutet.

Das in *para*-Stellung mit einem Halogenatom substituierte Benzylaminderivat stellt man besonders einfach in einer Gabriel-Reaktion unter für die Gabriel-Reaktion üblichen Bedingungen mit Phthalimid aus einem in *para*-Stettung mit einem Halogenatom, bevorzugt mit Brom substituierten Benzylhalogenid, bevorzugt *para*-Brombenzylbromid, her.

Bei der Syntheseroute B führt die Umsetzung der Verbindung der allgemeinen Formel IV mit der Verbindung der allgemeinen Formel V zu einer Verbindung der allgemeinen Formel I, wobei es sich bei dem Rest R¹ um einen Rest R^{1b} handelt.

In einer bevorzugten Ausführungsform wird die erhaltene Verbindung der allgemeinen Formel I mit einem Rest R^{1b} durch eine vorstehend beschriebene C-C-Kupplungsreaktion in eine Verbindung der allgemeinen Formel I mit einem Rest R^{1a} überführt.

Die Herstellung einer Verbindung der allgemeinen Formel I mit einem Rest R^{1a} erfolgt bevorzugt, indem man eine Verbindung der allgemeinen Formel I, in der R^{1b} Halogen, bevorzugt Bromid bedeutet, mit einer Verbindung der allgemeinen Formel III, in der R³ ein Rest der allgemeinen Formel VI, ein Trialkylzinnrest oder ein Magnesium(II)halogenidrest bedeutet, unter Bedingungen, wie sie für eine Suzuki-, Stille- oder Grignard-Reaktion üblich sind, umsetzt.

Syntheseroute C beschreibt den Fall, dass R⁵ in Verbindungen der allgemeinen Formel IV ein Rest der allgemeinen Formel VI bedeutet. (Das heißt, der Rest R⁵ in Formel IV entspricht den Rest R^{1b} in Formel I.)

Die Herstellung einer Verbindung der allgemeinen Formel IV, in der R⁵ ein Rest der allgemeinen Formel VI bedeutet, erfolgt bevorzugt, indem man ein in para-Stellung mit einem Rest R⁵ der allgemeinen Formel VI substituiertes Benzylaminderivat mit einer Verbindung der allgemeinen Formel VIII bevorzugt in Gegenwart einer Bronstedt-Base umsetzt.

Das in *para*-Stellung mit einem Rest R⁵ der allgemeinen Formel VI substituierte Benzylaminderivat wird im Allgemeinen in einer Gabriel-Reaktion unter für die Gabriel-Reaktion üblichen Bedingungen mit Phthalimid aus einem in *para*-Stellung mit einem Rest R⁵ der allgemeinen Formel VI substituierten Benzylhalogenid, bevorzugt mit einem Rest R⁵ der allgemeinen Formel VI substituierten Benzylbromid hergestellt.

Bei der Syntheseroute C führt die Umsetzung der Verbindung der allgemeinen Formel IV mit der Verbindung der allgemeinen Formel V zu einer Verbindung der allgemeinen Formel I, wobei es sich bei dem Rest R¹ um einen Rest R^{1b} handelt.

In einer bevorzugten Ausführungsform wird die erhaltene Verbindung der allgemeinen Formel I mit einem Rest R^{1b} durch eine vorstehend beschriebene C-C-Kupplungsreaktion in eine Verbindung der allgemeinen Formel I mit einem Rest R^{1a} überführt.

Die Verbindung der allgemeinen Formel I, in der R¹ ein Rest der allgemeinen Formel II bedeutet, wird nach einer besonders bevorzugten Methode hergestellt, indem man eine Verbindung der allgemeinen Formel I, in der R^{1b} ein Rest der allgemeinen Formel VI bedeutet, mit einer Verbindung der allgemeinen Formel III, in der R³ Halogen, bevorzugt Brom bedeutet, unter Bedingungen, wie sie für eine Suzuki-Reaktion üblich sind, umsetzt.

Für den Fall, dass in der allgemeinen Formel IV R⁵ ein Trialkylzinn oder MgHal-Rest ist, so ist ebenfalls ein Reaktionsverlauf wie in Syntheseroute C bevorzugt.

Ein weiterer Aspekt der Erfindung ist die Herstellung von einem Imidazolderivat, das an mindestens einem Kohlenstoffatom des Imidazolrings mit Chlor substituiert ist (imidazolderivat A), indem man Imidazol oder ein Imidazolderivat, das an mindestens einem Kohlenstoffatom des Imidazolrings ein Wasserstoffatom trägt (Imidazolderivat B), mit CeCl₃ und einem Alkalisalz einer Hypohalogensäure umsetzt.

Bevorzugt stellt man als Imidazolderivat (A) eine Verbindung her, die an dem Kohlenstoffatom des Imidazolrings in der 4- oder 5-Position oder an beiden vorgenannten Positionen mit Chlor substituiert ist, und wobei man als Imidazolderivat (B) eine Verbindung einsetzt, die an dem Kohlenstoffatom des Imidazolrings in der 4- oder 5-Position oder an beiden vorgenannten Positionen noch ein Wasserstoffatom trägt.

Ganz besonders eignet sich diese Chlorierungsmethode für die Herstellung von einem Losartan-Derivat, bei dem das Wasserstoffatom der Tetrazolgruppe durch eine Tetrazol-Schutzgruppe ersetzt ist, wobei man als Imidazolderivat (B) die Verbindung der allgemeinen Formel XI einsetzt.

Bevorzugt wird CeCl₃ und das Alkalisalz der Hypohalogensäure in stöchiometrischen Mengen oder im Überschuss eingesetzt. Mit Vorteil wird als Alkalisalz der Hypohalogensäure das Kalium- oder Natriumsalz eingesetzt. Bevorzugt wird als Alkalisalz der Hypohalogensäure ein Alkalisalz der hypochlorigen Säure eingesetzt.

Die erfindungsgemäße Chlorierungsreaktion wird üblicherweise in einem 2-phasigen System durchgeführt, bei dem eine Phase aus einer wässrigen Lösung gebildet ist und die andere Phase durch eine Lösung gebildet wird, die ein mit Wasser nicht unbegrenzt mischbares organisches Lösungsmittel wie Methylenchlorid, Chloroform oder Toluol umfasst.

Ausgehend von einer Verbindung der allgemeinen Formel I mit einem Rest R^{1a} lässt sich Losartan oder eines seiner pharmakologisch akzeptablen Salze besonders einfach herstellen, indem man
a) in einem Schritt (a) ausgehend von einer Verbindung der allgemeinen Formel I mit einem Rest R^{1a} die Verbindung der allgemeinen Formel XI 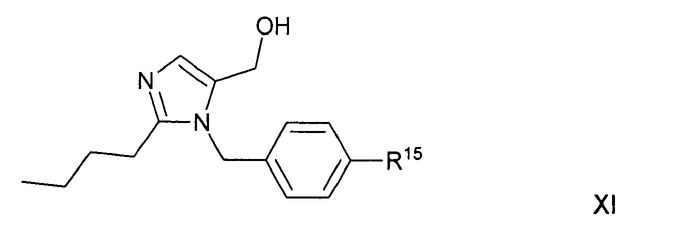 in der R¹⁵ ein Rest der allgemeinen Formel II bedeutet, herstellt, indem man die Formylgruppe, mit der die Imidazolgruppe substituiert ist, zu einer Hydroxymethylgruppe reduziert,
b) in einem Schritt (b) das einzige noch verbliebene Wasserstoffatom in der Imidazolgruppe der nach Schritt (a) hergestellten Verbindung durch Chlor ersetzt und
(c) in einem Schritt (c) in der nach Schritt (b) hergestellten Verbindung die Tetrazol-Schutzgruppe abspaltet und gegebenenfalls
(d) aus Losartan eines seiner pharmakologisch akzeptablen Salze, z.B. das Kalium-Salz herstellt.

Die Reduktion der Formylgruppe in Schritt (a) kann auf übliche Weise erfolgen. Bevorzugt erfolgt die Reduktion der Formylgruppe in Schritt (a) mit Natriumborhydrid oder Lithiumaluminiumhydrid.

Die Chlorierung in Schritt (b) kann auf übliche Weise erfolgen. Bevorzugt wird Schritt (b) unter Anwendung der oben beschriebenen Chlorierungsmethode, d.h. unter Verwendung von CeCl₃, ausgeführt.

Schritt (c) wird üblicherweise wie in WO 03/093262 beschrieben ausgeführt. Die Abspaltung der besonders bevorzugten Triphenylmethyl-Schutzgruppe gelingt z.B., indem man eine Lösung der nach Schritt (b) hergestellten Verbindung mit einer verdünnten Mineralsäure, bevorzugt Chlorwasserstoffsäure, behandelt.

Die Herstellung eines pharmakologisch akzeptablen Salzes von Losartan, z.B.Losartan-Kalium (Schritt d) wird z.B. wie in EP 324377 A, Seite 191, example 316, part D und WO 95/17396, Seite 18, example 4 und Seite 24, example 9, step C beschrieben vorgenommen.

Es werden nachfolgend die vorstehend beschriebenen Syntheserouten A, B und C näher erläutert.

Die in den jeweiligen Syntheserouten verwendeten und/oder entstehenden Verbindungen werden mit arabischen Ziffern bezeichnet. Dabei entsprechen die in den nachfolgenden Formelschemata dargestellten Verbindungen
- 4 einer Verbindung der allgemeinen Formel I mit einem Rest R¹ der allgemeinen Formel II (d.h. bei R¹ handelt es sich um einen Rest R^{1a}),
- 15 und 21 Verbindungen der allgemeinen Formel I, mit einem Rest R^{1b}
- 13, 23 Verbindungen der allgemeinen Formel III,
- 3, 14 Verbindungen der allgemeinen Formel IV,
- 8 einer Verbindung der allgemeinen Formel V,
- 21 einer Verbindung der allgemeinen Formel IV mit einem Rest R⁵ der allgemeinen Formel VI,
- 1 einer Verbindung der allgemeinen Formel VII,
- 2 einer Verbindung der allgemeinen Formel VIII, und
- 9, 10 Verbindungen der allgemeinen Formel IX.

Sie stellen zugleich bevorzugte Ausführungsbeispiele der durch die jeweilige allgemeine Formel definierten Verbindungsgruppe dar.

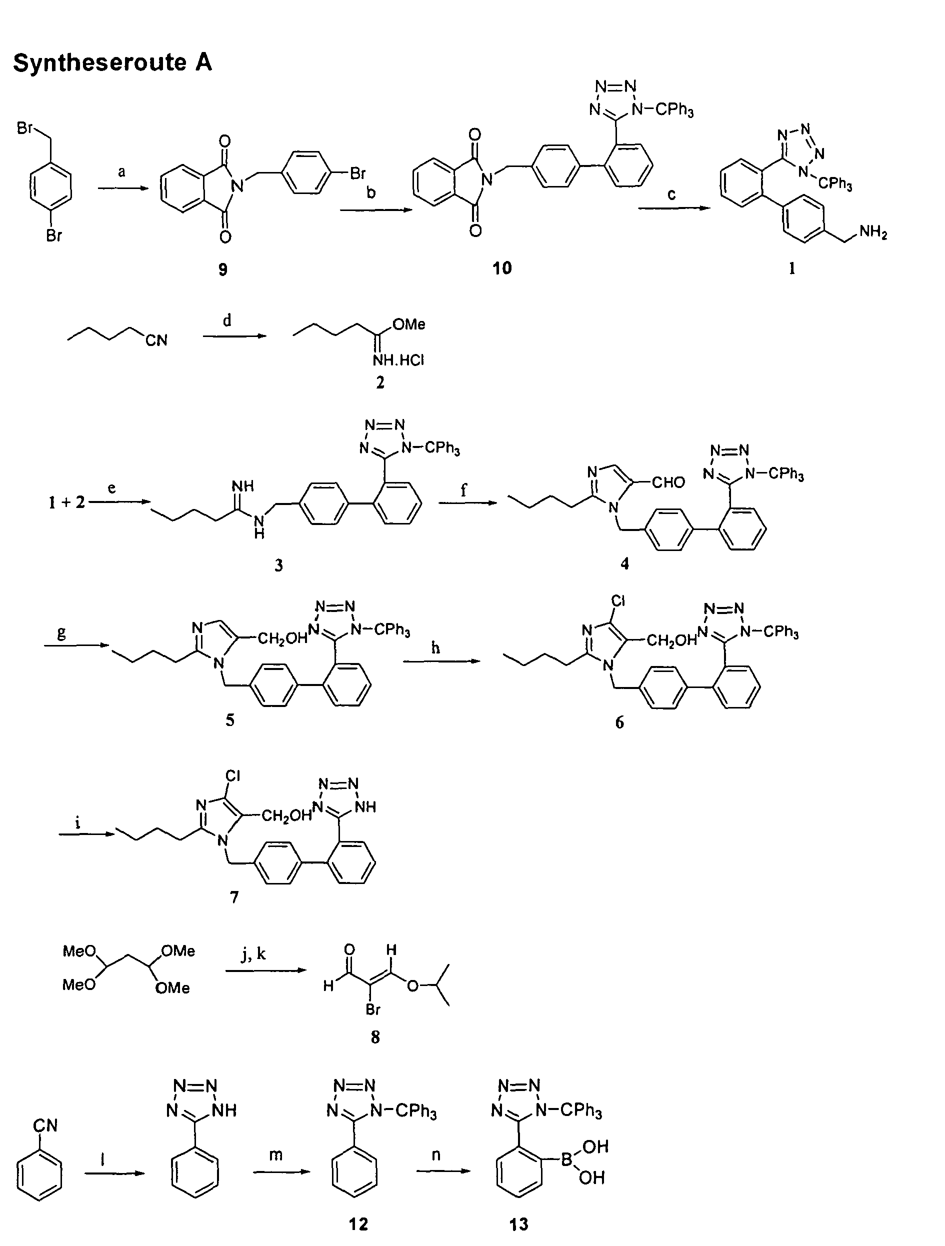

Die Umsetzungen a) bis n) erfolgen im Allgemeinen unter üblichen Reaktionsbedingungen, bevorzugt in Gegenwart folgender Reagenzien:
a) Phthalimid
b) **13**
d) CH₃OH
e) Base
f) **8**
g) Hydriermittel
h) Chlorierungsmittel

In einer besonders bevorzugten Ausführungsform können folgende Reagenzien und Reaktionsbedingungen verwendet werden:
a) Phthalimid, K₂CO₃ / DMSO;
b) **13,** Pd(PPh₃)₄, Na₂CO₃, Toluol-H₂O, 80 °C;
c) Hydrazinhydrat, CH₃OH / CH₂Cl₂;
d) CH₃OH / HCl;
e) NEt₃ / EtOH;
f) **8,** K₂CO₃ / CHCl₃-H₂O;
g) NaBH₄/CH₃OH;
h) CeCl₃.7H₂O, NaHlO/ CH₂Cl₂-H₂O;
i) 2N HCl, CH₃OH-THF;
j) konzentrierte HCl, Br₂;
k) PPTS, Isopropanol;
l) NaN₃,NH₄Cl, LiCl, DMF, 100 °C;
m) Ph₃CCl, Et₃N/CH₂Cl₂;
n) BuLi, -20 °C to -5 °C, dann B(OMe)₃, -20 °C bis R.T.

In einer bevorzugten Ausführungsform werden die Verbindungen **1** und **2** ohne Isolierung von Verbindung **3** zu Verbindung **4** umgesetzt.

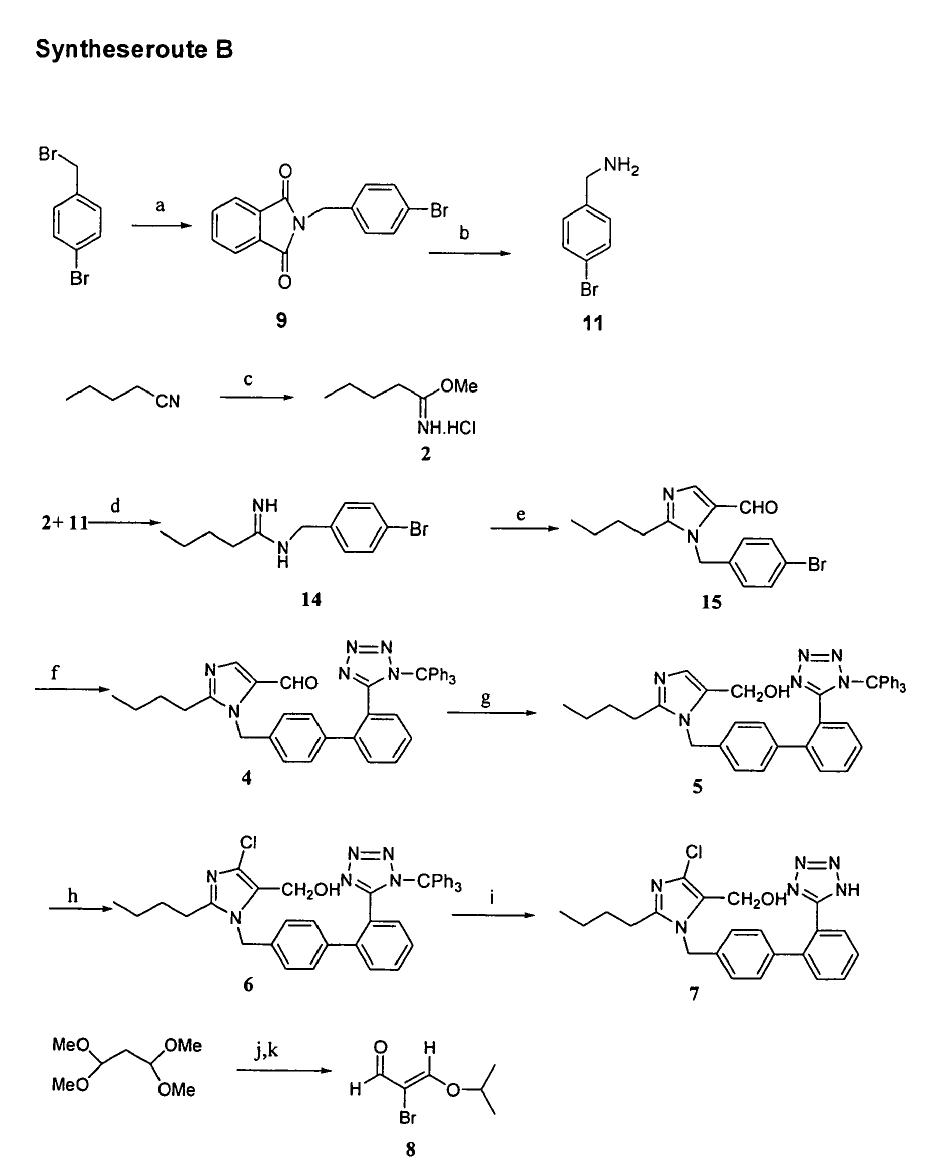

Die Umsetzungen a) bis k) erfolgen im Allgemeinen unter üblichen Reaktionsbedingungen, bevorzugt in Gegenwart folgender Reagenzien:
a) Phthalimid
c) CH₃OH
d) Base
e) **8**
f) **13**
g) Hydriermittel
h) Chlorierungsmittel

In einer besonders bevorzugten Ausführungsform können folgende Reagenzien und Reaktionsbedingungen verwendet werden:
a) Phthalimid, K₂CO₃ / DMSO;
b) Hydrazinhydrat, CH₃CH₂OH/H₂O;
c) CH₃OH/HCl;
d) NEt₃/EtOH;
e) **8**, K₂CO₃/CHCl₃-H₂O;
f) **13,** Pd(PPh₃)₄, Na₂CO₃, Toluol-H₂O, 80 °C;
g) NaBH₄/CH₃OH;
h) CeCl₃.7H₂O, NaClO/CH₂Cl₂-H₂O;
i) 2N HCl, CH₃OH-THF;
j) konzentrierte HCl, Br₂;
k) PPTS, Isopropanol;

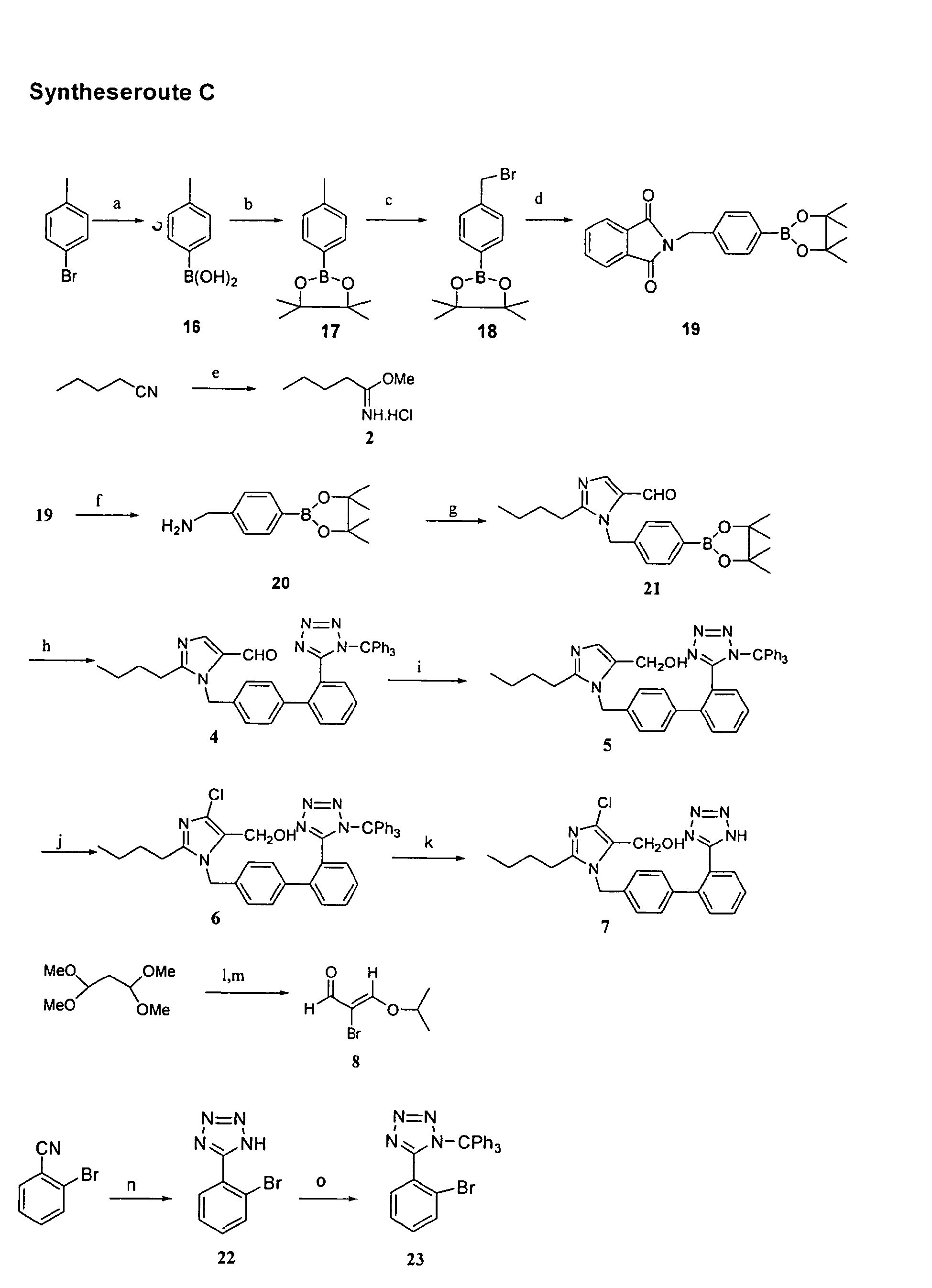

Die Umsetzungen a) bis o) erfolgen im Allgemeinen unter üblichen Reaktionsbedingungen, bevorzugt in Gegenwart folgender Reagenzien:
d) Phthalimid
e) CH₃OH
g) **2**, Base, dann 8
h) **13**
i) Hydriermittel
j) Chlorierungsmittel

In einer besonders bevorzugten Ausführungsform können folgende Reagenzien und Reaktionsbedingungen verwendet werden:
a) Mg, I₂, THF, Rückfluss 1 h, dann -78 °C, B(OMe)₃;
b) Pinacol, Cyclohexan, Rückfluss zum Wasser entfernen;
c) NBS, Cyclohexan, Rückfluss;
d) Phthalimid, K₂CO_{3/}Aceton, Rückfluss;
e) CH₃OH/HCl;
f) Hydrazinhydrat, CH₃CH₂OH, Rückfluss;
g) **2**, NEt₃/CH₃OH, dann K₂CO₃, **8**;
h) **13**, Pd(PPh₃)₄, K₂CO₃, Toluol-H₂O, 80 °C;
i) NaBH₄/CH₃OH;
j) CeCl₃.7H₂O, NaClO/CH₂Cl₂-H₂O;
k) 2N HCl, CH₃OH-THF;
l) konzentrierte HCl, Br₂;
m) PPTS, Isopropranol;
n) NaN₃, NH₄Cl, LiCl, DMF, 100 °C;
o) Ph₃CCl, Et₃N/CH₂Cl₂.

### Experimenteller Teil:

Im experimentellen Teil wird die Herstellung der in den Syntheserouten A, B oder C vorkommenden Verbindungen näher beschrieben.

### Versuchsbeschreibungen

### Geräte und Reagenzien

Alle trockenen Lösungsmittel (CH₂Cl₂, THF, Et₂O, Benzol, Toluol, DMF, MeCN) wurden nach Standardmethoden getrocknet, d.h. durch Entfernung von Wasser und Sauerstoff und Destillation vor Benutzung. Die Reaktionen wurden, insofern nötig, in einer Inertgasatmosphäre (N₂ oder Ar) durchgeführt und überwacht mit Hilfe der TLC. Die Lösungsmittel für die Extraktionen waren beispielsweise Diethylether, Ethylacetat oder Chloroform. Die Extrakte wurden, soweit nicht anders angegeben, beispielsweise mit wasserfreiem MgSO₄ getrocknet. Die Reaktionsprodukte wurden, insofern notwendig, beispielsweise mit Hilfe der Säulenchromatographie unter Verwendung von beispielsweise Petrolether (60 - 90 °C) / Ethylacetat und Chloroform / Methanol als Eluent gereinigt. Falls Platten des Typs GF₂₅₄ für die TLC benutzt wurden, wurde als Detektionsmittel lod oder eine ethanolische Lösung von Phosphormolybdänsäure verwendet. Das Silicagel für die Chromatographie (200 - 300 Körnung) und TLC (GF₂₅₄) wurde hergestellt von *Qingdao Sea Chemical Factory* und *Yantan Chemical Factory.* Alle Lösungsmittel und Reagenzien waren von analytischer oder chemischer Reinheit. Die Schmelzpunkte wurden bestimmt mit einem XT₄-100x *micro-melting point tester.* Nicolet AVATAR 360 FT-IR und Nicolet NEXUS 670 FT-IR Spektrometer wurden zur Aufnahme von Infrarotspektren mit KBr-Presslingen oder PE-Filmen verwendet. Mercury-300 (Varian) und AM-400 (Bruker) Spektrometer wurden für NMR Messungen mit SiMe₄ als internem Standard und CDCl₃ als Lösungsmittel, soweit nicht anders vermerkt, verwendet. LRMS wurden bestimmt mit einem HP-5988 Massenspektrometer unter Verwendung von EI bei 70eV, soweit nicht anders angegeben. HRMS wurden gemessen mit einem Bruker Daltonics APEX II 47e FT-ICR Massenspektrometer.

### Darstellung von Verbindung 9

Phthalimid (11 g, 75,6 mmol) wurde in 80 ml DMSO unter Argon Schutzgasatmosphäre gelöst. Nach Zugabe von K₂CO₃ (20 g, 144 mmol) wurde die Reaktionsmischung 2 h auf 120 °C erhitzt. Anschließend wurde die Reaktionsmischung auf etwa 50 °C abgekühlt und *p*-Brombenzylbromid (18 g, 72 mmol) zugegeben. Nach weiteren 10 h Rühren wurden 100 ml H₂O zugegeben. Der farblose Niederschlag wurde abfiltriert, gewaschen und getrocknet, was Verbindung 9 (18.6 g) ergab. Die Ausbeute betrug 82%.
¹H NMR (CDCl₃, 300 MHz): δ 4,77 (s, 2H, NCH₂), 7,29 (d, *J*=8,4 Hz, 2H, ArH), 7,41 (d, *J*=8,4 Hz, 2H, ArH), 7,67-7,70 (m, 2H, ArH), 7,80-7,83 (m, 2H, ArH); ¹³C NMR (CDCl₃, 75 MHz): δ 40,9, 121,8, 123,3 (2C), 130,3 (2C), 131,7 (2C), 131,9 (2C), 134,0 (2C), 135,2, 167,8 (2C); MS (FAB): M⁺= 315, gefunden: 316 (M⁺+1), 318 (M⁺+3); IR (Film, cm-¹) *v*ₘₐₓ=3460, 3100, 3045, 2938, 1771, 1702, 1612, 1486, 1464, 1430, 1399, 1332, 1298, 1173, 1077, 1010, 957, 935, 845, 796, 731, 713, 528.

### Darstellung von Verbindung 10

Verbindung **9** (1,45 g, 4,6 mmol), Verbindung **13** (3,4 g, 1,2 Äquivalente) und Na₂CO₃ (1,46 g, 3 Äquivalente) wurden in einer Mischung von 20 ml Toluol/H₂O (7:3) gelöst. Anschließend wurde das System 3-fach mit Argon gespült und Pd(PPh₃)₄ (266 mg, 0,05 Äquivalente) zugegeben. Die Reaktionsmischung wurde 13 h auf 80 °C erwärmt und anschließend mit Ethylacetat extrahiert. Die organischen Phasen wurden eingeengt und der Feststoff mittels Säulenchromatographie gereinigt, was Verbindung 10 als weißen Feststoff (2,43 g) ergab. Die Ausbeute betrug 86%.
¹H NMR (CDCl₃, 300 MHz): δ 4,73 (s, 2H), 6,87-6,90 (m, 6H), 7,06 (d, J=8,1 Hz, 2H), 7,18-7,33 (m, 12H), 7,42-7,46 (m, 2H), 7,67-7,70 (m, 2H), 7,82-7,84 (m, 2H), 7,92-7,95 (m, 1 H); ¹³C NMR (CDCl₃, 75 MHz): δ 41,2. 82,8, 123,3, 126,2, 127,6, 128,2, 129,5, 129,8, 130,2, 130,7, 132,1, 133,9, 134,8, 140,7, 141,2, 141,7, 163,9, 167,9; MS (FAB): M⁺ = 623, gefunden: 662 (M⁺ + K) ; IR (Film, cm⁻¹) *v*ₘₐₓ = 3467, 3061, 3032, 2249, 1770, 1714, 1603, 1492, 1469, 1446, 1429, 1393, 1349, 1187, 1159, 1087, 1031, 1004, 937, 909, 880, 733, 700, 633, 406.

### Darstellung von Verbindung 1

Verbindung **10** (37,3 g, 60 mmol) wurde in einer Mischung von 200 ml Methanol und 300 ml CH₂Cl₂ gelöst. Hydrazinhydrat (600 mmol, 10 Äquivalente) wurde hinzugegeben und die Reaktionsmischung 10 h bei Raumtemperatur gerührt. Anschließend wurde filtriert und das Filtrat mit CHCl₃ verdünnt und mit Wasser gewaschen. Die organische Phase wurde über MgSO₄ getrocknet und eingeengt was Verbindung **1** als gelblichen Feststoff (23,7 g) ergab. Die Ausbeute betrug 80%.
¹H NMR (CDCl₃, 200 MHz): δ 3,80 (s, 2H), 4,52 (s, br, 2H), 6,89-6,92 (m, 6H), 7,04-7,14 (m, 4H), 7,24-7,31 (m, 10H), 7,43 (s, br, 2H), 7,90-7,93 (m, 1 H); MS (FAB): M⁺ = 493, gefunden: 494 (M⁺ + 1), 516 (M⁺ + Na).

### Darstellung von Verbindung 2

HCl-Gas wurde in eine Lösung von 8,3 g (100 mmol) Valeronitril in 8 ml Methanol unter Eisbad-Kühlung eingeleitet. Die Temperatur wurde dabei stets unter 10 °C gehalten. Nach 2 h war die Reaktion beendet und Verbindung 2 als weißer Feststoff erhalten.
¹H NMR (CDCl₃, 300 MHz): δ 0,84 (t, J=7,2 Hz, 3H, CH₃), 1,26-1,34 (m, 2H, CH₂), 1,57-1,67 (m, 2H, CH₂), 2,68 (t, J=7,5Hz, 2H, CH₂), 4,19 (s, 3H, OMe), 7,43 (s, 1H, NH), 11,12 (s, br, 1H, HCl), ¹³C NMR (CDCl₃, 75MHz) : δ 13,2, 21,7, 27,3, 32,5, 60,4, 180,6.

### Darstellung von Verbindung 8

Konzentrierte HCl (3,6 ml) wurde in eine Mischung von 1,1,3,3-Tetramethoxypropan (6,5 g, 40 mmol) und Wasser (64 ml) getropft. Die Reaktionsmischung wurde durch rühren homogenisiert, auf 0 °C abgekühlt und tropfenweise mit Brom (2,1 ml, 40 mmol) versetzt. Es wurde weitere 10 min gerührt und anschließend ein Großteil des Wassers im Vakuum bei unter 70 °C entfernt. Nach erneutem Abkühlen auf 0 °C wurde filtriert. Der so erhaltene Feststoff wurde getrocknet und anschließend in einer Mischung von 65 ml Cyclohexan und 10 ml Isopropanol gelöst und es wurde eine katalytische Menge PPTS hinzugegeben. Die Reaktionsmischung wurde 90 min unter Rückfluss erhitzt und das entstehende Wasser abgetrennt. Die übrigen Lösungsmittel wurden im Vakuum entfernt. Verbindung 8 blieb als gelbes Öl zurück. Die Reinheit war größer 95%, so dass Verbindung 8 direkt ohne weitere Reinigung eingesetzt werden kann. Die Ausbeute betrug 65%.
¹H NMR (CDCl₃, 300 MHz): δ 1,38 (d, *J*=6 Hz, 6H, 2CH₃), 4,45-4,50 (m, 1 H, CH), 7,70 (s, 1 H, CH=), 9,07 (s, 1 H, CHO); ¹³C NMR (CDCl₃, 75 MHz): δ 22,3 (2C), 80,6, 105,1, 166,2, 184,0; MS (EI) *m*/*z* (%):194 (M⁺, 5), 192 (M⁺, 5), 166 (2), 152 (95), 150 (100), 121 (13), 93 (16), 71 (30), 43 (59).

### Darstellung von Verbindung 3

Verbindung **2** (6,4 g, 40 mmol) wurde in 75 ml absolutem Ethanol und 20 ml NEt₃ gelöst. Dann wurde Verbindung **1** (10 g, 20 mmol) bei 10 °C zugegeben und die Reaktionsmischung 5 h gerührt, was eine Lösung ergab, die weitere 8h bei Raumtemperatur gerührt wurde. Anschließend wurde mit Chloroform verdünnt und mit Wasser gewaschen. Die organische Phase wurde abgetrennt, getrocknet und eingeengt bei einer Temperatur unterhalb von 45 °C. Verbindung 3 wurde als Öl erhalten. Die Ausbeute betrug 80%.
¹H NMR (CDCl₃, 300 MHz): δ 0,67-0,80 (m, 3H, CH₃), 1,09-1,24 (m, 2H, CH₂), 1,58 (s, br, 2H, CH₂), 2,51 (s, br, 2H, CH₂), 4, 53 (s, br, 2H, NCH₂), 6,89-7,08 (m, 8H, ArH), 7,21-7,44 (m, 14H, ArH), 7,87 (d, J=3,9Hz, 1 H, ArH); ¹³C NMR (CDCl₃, 75 MHz): δ 13,3, 21,6, 28,0, 32,3, 45,4, 82,9, 125,5, 126,0, 127,5, 128,2, 129,2, 129,8, 133,3, 140,3, 140,7, 141,0, 163,9, 167,7; MS (FAB): M⁺= 576, gefunden: 577 (M⁺ + 1) ; IR (Film, cm-¹) *v*ₘₐₓ = 3222, 3057, 3030, 2962, 2933, 2210, 1677, 1636, 1531, 1493, 1449, 1190, 1004, 910, 732, 700, 639.

### Darstellung von Verbindung 4

### Methode A-1 (mit Isolierung von Verbindung 3):

Verbindung **3** (8 g, 13,9 mmol) wurde in 60 ml Chloroform und 7,5 ml Wasser gelöst. Nach Zugabe von K₂CO₃ (2,69 g, 19,5 mmol) und Verbindung 8 (3,73 g, 19,5 mmol) wurde die Reaktionsmischung 12 h bei Raumtemperatur gerührt. Anschließend wurde mit Chloroform extrahiert und die organische Phase eingeengt. Das so erhaltene Rohprodukt wurde mittels Säulenchromatographie gereinigt was Verbindung **4** als weißen Feststoff (3.67 g) ergab. Die Ausbeute betrug 42%.

### Methode A-2 (ohne Isolierung von Verbindung 3):

Verbindung **2** (4,5 g, 1,5 Äquivalente, 30 mmol) wurde in einer Mischung von 50 ml absolutem Ethanol und NEt₃ (8,3 ml, 3 Äquivalente) bei 0 °C gelöst und Verbindung 1 (10 g, 1 Äquivalent, 20 mmol) wurde hinzugefügt. Die Reaktionsmischung wurde etwa 5 h gerührt um eine klare Lösung zu erhalten und weitere 16 h bei Raumtemperatur. Anschließend wurden K₂CO₃ (4,14 g, 30 mmol, 1,5 Äquivalente) und Verbindung 8 (4,6 g, 1,2 Äquivalente, 24 mmol) zugegeben und weitere 12 h bei Raumtemperatur gerührt. Die Reaktionsmischung wurde extrahiert mit CHCl₃ und die organische Phase eingeengt. Der erhaltene Feststoff wurde mittels Säulenchromatographie gereinigt was Verbindung **4** als weißen Feststoff ergab.

### Methode A-3 (ohne Isolierung von Verbindung 3):

Verbindung **1** (563 g) wurde in einer Mischung von 3,6 I absolutem Ethanol und 1,2 I Triethylamin gelöst. Die Reaktionsmischung wurde auf 0°C abgekühlt und Verbindung **2** (350 g) wurde langsam zugegeben. Es wurde 1 h bei 0°C gerührt und anschließend die Reaktionsmischung mit Chloroform und Wasser verdünnt. Die organische Phase wurde abgetrennt und die wässrige Phase wurde erneut mit Chloroform extrahiert. Zu den vereinigten organischen Phasen wurden bei Raumtemperatur K₂CO₃ (180 g), 560 ml Wasser und 330 g Verbindung 8 gegeben. Anschließend wurde über Nacht bei Raumtemperatur gerührt und die Reaktionsmischung mit Chloroform und Wasser verdünnt. Die organische Phase wurde abgetrennt und die wässrige erneut mit Chloroform extrahiert. Die vereinigten organischen Phasen wurden mittels MgSO₄ getrocknet, filtriert und eingeengt. Der so erhaltene Rückstand wurde aus Ethylacetat umkristallisiert, was Verbindung **4** ergab (530 g). Die Ausbeute betrug 74%

### Methode B:

Verbindung **15** (1,47 g, 4,6 mmol), Verbindung **13** (3,4 g, 1,2 Äquivalente) und Na₂CO₃ (1,46 g, 3 Äquivalente) wurden in 20 ml einer Mischung von Toluol und Wasser (7:3) gelöst. Anschließend wurde das System 3-fach mit Argon gespült und Pd(PPh₃)₄ (266 mg, 0,05 Äquivalente) zugegeben. Die Reaktionsmischung wurde 10 h auf 80 °C erwärmt und anschließend mit Ethylacetat extrahiert. Die organischen Phasen wurden eingeengt und der Rückstand mittels Säulenchromatographie gereinigt, was Verbindung **4** als Feststoff (2,1 g) ergab. Die Ausbeute betrug 74%.

### Methode C:

Verbindung **21** (40 mg, 0,11 mmol), Verbindung **23** (151 mg, 0,33 mmol) und K₂CO₃ (45 mg, 0,33 mmol) wurden in 3 ml einer Mischung von Toluol und Wasser (7:3) gelöst. Anschließend wurde das System 3-fach mit Argon gespült und Pd(PPh₃)₄ (6 mg, 0,05 Äquivalente) zugegeben. Die Reaktionsmischung wurde 10 h auf 80 °C erwärmt und anschließend mit Ethylacetat extrahiert. Die organischen Phasen wurden eingeengt und der Rückstand mittels Säulenchromatographie gereinigt, was Verbindung **4** als Feststoff (48 mg) ergab. Die Ausbeute betrug 72%.
¹H NMR (CDCl₃, 400 MHz,): δ 0,86 (t, *J*= 7,2 Hz, 3H, CH₃), 1,25-1,32 (m, 2H, CH₂), 1,64-1,68 (m, 2H, CH₂), 2,55 (t, *J*= 7,6 Hz, 2H, CH₂), 5,48 (s, 2H, NCH₂), 6,82 (d, *J*= 8,4 Hz, 2H, ArH), 6,91-6,93 (m, 6H, ArH), 7,09 (d, *J*= 8,4 Hz, 2H, ArH), 7,23-7,27 (m, 6H, ArH), 7,31-7,35 (m, 4H, ArH), 7,41-7,49 (m, 2H, ArH), 7,78 (s, 1 H, CH=), 7,91 (dd, *J*= 1,2 Hz, 7,2 Hz, ArH), 9,64 (s, 1H, CHO); ¹³C NMR (CDCl₃, 100 MHz,): δ 13,7, 22,3, 26,4, 29,2, 47,8, 82,8, 125,9, 126,2, 127,6, 127,8, 127,9, 128,2, 129,7, 129,9, 130,1, 130,2, 130,7, 131,3, 134,7, 140,7, 141,2, 141,4, 143,6, 156,7, 163,8, 178,5; MS (FAB): M⁺ = 628, gefunden: 629 (M⁺ +1), 651 (M⁺ + Na); IR (Film, cm-¹) *v*ₘₐₓ = 3060, 3031, 2959, 2932, 2868, 1670, 1619, 1597, 1532, 1466, 1446, 1187, 1160, 1030, 1003, 909, 880, 824, 762, 733, 701,640.

### Darstellung von Verbindung 5

Verbindung **4** (6,3 g, 10 mmol) wurde in 30 ml Methanol suspendiert und es wurden 3 ml CHCl₃ zugegeben zum vollständigen Auflösen. Die Reaktionsmischung wurde in einem Eisbad gekühlt und NaBH₄ (760 mg, 20 mmol) zugegeben. Nach 1 h rühren wurde die Mischung mit CHCl₃ extrahiert. Die organische Phase wurde eingeengt was Verbindung 5 (6 g) als Öl ergab. Die Ausbeute betrug 95%.
¹H NMR (CDCl₃, 300 MHz): δ 0,85 (t, *J*= 7,5 Hz, 3H, CH₃), 1,27-1,32 (m, 2H, CH₂), 1,61-1,66 (m, 2H, CH₂), 2,50 (t, *J*= 7,5Hz, 2H, CH₂), 4,32 (s, 2H, CH₂OH), 5,12 (s, 2H, NCH₂), 6,74 (d, *J*= 8,1 Hz, 2H, ArH), 6,84 (s, 1H, CH=), 6,93 (d, *J*= 7,2 Hz, 6H, ArH), 7,08 (d, *J*= 8,1 Hz, 2H, ArH), 7,23-7,36 (m, 10H, ArH), 7,44-7,48 (m, 2H, ArH), 7,90-7,93 (m, 1H, ArH); ¹³C NMR (CDCl₃, 75 MHz): δ 13,7, 22,3, 26,7, 29,7, 46,3, 54,4, 82,8, 125,2, 126,2, 126,6, 127,6, 128,2, 129,7, 130,2, 130,7, 131,0, 135,3, 140,5, 141,2, 141,4, 150,1, 163,9; MS (FAB): M⁺ = 630, gefunden: 631 (M⁺+1), 653 (M⁺ + Na); IR (Film, cm⁻¹) *v*ₘₐₓ = 3060, 2956, 2927, 2865, 1493, 1464, 1448, 1355, 1272, 1189, 1153, 1026, 906, 880, 822, 753, 699, 636.

### Darstellung von Verbindung 6

Verbindung **5** (6,3 g, 10 mmol) wurde in einem Lösungsmittelgemisch von 40 ml CH₂Cl₂ und Wasser (1:1) gelöst. Nach Zugabe von CeCl₃.7H₂O (7,44 g, 20 mmol) und weiterem 2-minütigem Rühren wurde eine 10%ige wässrige Lösung von NaClO (37 ml) zugetropft. Anschließend wurde weitere 10 min gerührt und mit einer gesättigten wässrigen Lösung von Na₂SO₃ versetzt. Die Reaktionsmischung wurde extrahiert mit CHCl₃, die organischen Phasen eingeengt und der erhaltene Feststoff mittels Säulenchromatographie gereinigt was Verbindung 6 (4,65 g) ergab. Die Ausbeute betrug 70%.
¹H NMR (CDCl₃, 300 MHz): δ 0,86 (t, *J*= 7,2 Hz, 3H, CH₃), 1,23-1,33 (m, 2H, CH₂), 1,58-1,69 (m, 2H, CH₂), 2,49 (t, *J*= 7,8Hz, 2H, CH₂), 3,30 (s, br, 1H, OH), 4,32 (s, 2H, CH₂OH), 5,14 (s, 2H, NCH₂), 6,78 (d, *J*= 7,8 Hz, 2H, ArH), 6,94 (d, *J*= 7,5 Hz, 6H, ArH), 7,12 (d, *J*= 7,8 Hz, 2H, ArH), 7,23-7,37 (m, 10H, ArH), 7,43-7,51 (m, 2H, ArH), 7,94-7,97 (m, 1H, ArH); ¹³C NMR (CDCl₃, 75 MHz): δ 13,6, 22,3, 26,5, 29,5, 47,0, 52,7, 82,8, 124,9, 125,2, 126,1, 126,8, 127,5, 128,2, 129,7, 130,1, 130,6, 134,5, 140,7, 141,1, 141,2, 148,3, 163,8; MS (FAB): M⁺ = 664, gefunden: 665 (M⁺ +1), 687 (M⁺ + Na); IR (Film, cm-¹) *v*ₘₐₓ = 3184, 3061, 2958, 2931, 2869, 2244, 1577, 1492, 1466, 1447, 1356, 1255, 1189, 1160, 1078, 1028, 1005, 909, 881, 756, 733, 701, 640.

### Darstellung von Verbindung 7

Verbindung 6 (6,64 g, 10 mmol) wurde in 20 ml THF gelöst und mit 20 ml 2N HCl versetzt. Die Reaktionsmischung wurde bei Raumtemperatur 4 h gerührt, anschließend mit CHCl₃ verdünnt, mit Wasser gewaschen und getrocknet. Die organischen Phasen wurden eingeengt und der erhaltene Feststoff mittels Säulenchromatographie gereinigt was Verbindung 7 (3,8 g) ergab. Die Ausbeute betrug 90%.
¹H NMR (d-DMSO, 300 MHz): δ 0,78 (t, *J*= 7,2 Hz, 3H, CH₃), 1,18-1,25 (m, 2H, CH₂), 1,41-1,46 (m, 2H, CH₂), 2,44 (t, *J*= 7,5 Hz, 2H, CH₂), 4,32 (s, 2H, CH₂OH), 5,23 (s, 2H, NCH₂), 7,01 (d, *J*= 8,1 Hz, 2H, ArH), 7,07 (d, *J*= 8,1 Hz, 2H, ArH), 7,49-7,58 (m, 2H, ArH), 7,63-7,65 (m, 2H, ArH); ¹³C NMR (d-DMSO, 75 MHz): δ 13,5, 21,5, 25,7, 28,9, 46,4, 51,3, 123,5, 125,2, 125,6, 126,2 (2C), 127,7, 129,1 (2C), 130,5 (2C), 131,0, 136,1, 138,4, 141,0, 147,3, 155,0; MS (FAB): M⁺ = 422, gefunden: 423 (M⁺+1), 445 (M⁺ + Na); IR (Film, cm⁻¹) *v*ₘₐₓ= 3351, 2959, 2932, 2870, 1936, 1709, 1575, 1464, 1422, 1361, 1257, 1226, 1078, 1007, 824, 758.

### Darstellung von Verbindung 11

Verbindung **9** (9,5 g, 30 mmol) wurde in einer Mischung von 100 ml Ethanol und 30 ml Wasser gelöst. Anschließend wurde Hydrazinhydrat (9 ml) hinzugegeben und auf Rückfluss erhitzt. Nach etwa einer Stunde scheidet sich ein weißer Feststoff ab und nach weiteren 9 h Rühren unter Rückfluss wurde auf Raumtemperatur abgekühlt. Eine NaOH Lösung (4.88 M, 100 ml) wurde hinzugefügt und die Reaktionsmischung mit Diethylether extrahiert. Die organische Phase wurde über MgSO₄ getrocknet und eingeengt, was Verbindung 11 (5 g) ergab. Die Ausbeute betrug 90%.
¹H NMR (CDCl₃, 300 MHz) : δ 1,36 (s, 2H, NH₂), 3,75 (s, 2H, NCH₂), 7,12 (d, J=8,1 Hz, 2H, ArH), 7,38 (d, *J*=8,1 Hz, 2H, ArH); ¹³C NMR (CDCl₃, 75 MHz): δ 45,5, 120,2, 128,6 (2C), 131,2 (2C), 142,0; IR (Film, cm⁻¹) *v*ₘₐₓ = 3380, 2924, 2854, 2645, 2210, 1653, 1562, 1529, 1481, 1441, 1410, 1380, 1332, 1072, 1007, 905,812,789,645,618.

### Darstellung von Verbindung 15

Verbindung 2 (6,8 g, 1,5 Äquivalente, 45 mmol) wurde in einer Mischung von 60 ml absolutem Ethanol und NEt₃ (12,5 ml, 3 Äquivalente) bei 0 °C gelöst und anschließend Verbindung **11** (5,6 g, 1 Äquivalent, 30 mmol) hinzugegeben. Die Reaktionsmischung wurde bei Raumtemperatur 10 h gerührt und dann mit K₂CO₃ (6,2 g, 45 mmol, 1,5 Äquivalente) und Verbindung 8 (6,9 g, 1,3 Äquivalente, 36 mmol) versetzt und weitere 12 h bei Raumtemperatur gerührt. Anschließend wurde mit CHCl₃ extrahiert, die organische Phasen eingeengt und der Rückstand mittels Säulenchromatographie gereinigt, was Verbindung 15 (3,74 g) ergab. Die Ausbeute betrug 39%.
¹H NMR (CDCl₃, 300 MHz): δ 0,88 (t, *J*= 7,2 Hz, 3H, CH₃), 1,31-1,38 (m, 2H, CH₂), 1,63-1,71 (m, 2H, CH₂), 2,63 (t, *J*= 8,1 Hz, 2H, CH₂), 5,50 (s, 2H, NCH₂), 6,88 (d, *J*= 8,4 Hz, 2H, ArH), 7,42 (d, *J*= 8,4 Hz, 2H, ArH), 7,77 (s, 1H, CH=), 9,64 (s, 1H, CHO); ¹³C NMR (CDCl₃, 75 MHz): δ 13,6, 22,4, 26,4, 29,3, 47,5, 121,7, 128,0 (2C), 131,2, 131,9 (2C), 135,2, 143,7, 156,6, 178,7; MS (FAB): M⁺ = 320, gefunden: 321 (M⁺ +1), 323 (M⁺ + 3); IR (Film, cm⁻¹) *v*ₘₐₓ= 2958, 2932, 2868, 1671, 1533, 1485, 1463, 1407, 1373, 1161, 1072, 1011, 813, 769, 648.

### Darstellung von Verbindung 16

Eine Lösung von p-Bromtoluol (17 g, 100 mmol) in 100 ml getrocknetem THF wurde zu einer Mischung von Magnesium Pulver (3,6 g, 150 mmol), Iod (200 mg) und 4 Tropfen 1,2-Dibromethan innerhalb einer Stunde zugetropft. Anschließend wurde eine Stunde unter Rückfluss erhitzt und danach auf -78 °C abgekühlt und Trimethylborat (10,3 g 10 mmol) zugegeben. Die Reaktionsmischung wurde weitere 2 h gerührt und anschließend durch Zugabe von Wasser gequentscht. Nach Extraktion mit Ethylacetat wurde die organische Phase mit Wasser gewaschen, getrocknet und eingeengt. Der Rückstand wurde mittels Säulenchromatographie gereinigt, was Verbindung **16** (10,2 g) als farblose Kristalle ergab. Die Ausbeute betrug 75%.
¹H NMR (CDCl₃, 300 MHz): δ 2,41 (s, 3H, CH₃), 7,28 (d, *J*= 7,5 Hz, 2H, ArH), 8,09 (d, *J*= 7,5 Hz, 2H, ArH); ¹³C NMR (CDCl₃, 75 MHz): δ 21,9, 128,8 (2C), 135,7 (3C), 142,9; MS (EI): m/z (%): 354 (M⁺, 100), 262 (19), 193 (17), 145 (18), 119 (36), 91 (39), 43 (47); IR (Film, cm⁻¹) *v*ₘₐₓ = 3045, 3022, 2918, 1920, 1613, 1517, 1406, 1367, 1347, 1307, 1179, 1109, 1081, 818, 736, 685, 528, 477.

### Darstellung von Verbindung 17

Trimerisierte Borsäure **16** (5 g, 14,1 mmol), Pinacolhexahydrat (11,5 g, 50,8 mmol) wurden in 100 ml Cyclohexan gelöst und die Lösung zur Wasserentfernung 10 h auf Rückfluss erhitzt. Anschließend wurde das Cyclohexan unter vermindertem Druck abdestilliert und der Rückstand mittels Säulenchromatographie gereinigt, was Verbindung **17** (7,8 g) als Öl ergab. Die Ausbeute betrug 84%.
¹H NMR (CDCl₃, 300 MHz): δ 1,37 (s, 12H, 4CH₃), 2,40 (s, 3H, CH₃), 7,22 (d, *J*= 7,5 Hz, 2H, Ar), 7,75 (d, *J*= 7,5 Hz, 2H, Ar); ¹³C NMR (CDCl₃, 75 MHz): δ 21,7, 24,8 (4C), 83,5 (2C), 128,5 (2C), 134,8 (3C), 141,3; MS (EI): *m*/*z* (%): 218 (M⁺, 23), 203 (33), 132 (52), 119 (100), 91 (22), 43 (58); IR (Film, cm⁻¹) *v*ₘₐₓ = 3046, 2979, 2928, 1613, 1519, 1448, 1398, 1361, 1320, 1268, 1214, 1146, 1089, 1023, 962, 859, 816, 726, 656.

### Darstellung von Verbindung 18

Verbindung **17** (5 g, 22,9 mmol), NBS (5,3 g, 29,8 mmol) und AIBN (200 mg) wurden in 40 ml Cyclohexan gelöst und die Lösung 5,5 h auf Rückfluss erhitzt. Anschließend wurde unter vermindertem Druck filtriert und das Filtrat eingeengt. Der Rückstand wurde mittels Säulenchromatographie gereinigt, was Verbindung **17** (5,86 g) als Öl ergab. Die Ausbeute betrug 86%.
¹H NMR (CDCl₃, 300 MHz): δ 1,35 (s, 12H, 4CH₃), 4,45 (s, 2H, CH₂), 7,40 (d, J= 7,2 Hz, 2H, ArH), 7,81 (d, *J*= 7,2 Hz, 2H, ArH); ¹³C NMR (CDCl₃, 75 MHz): δ 24,8 (4C), 33,2, 83,8 (2C), 125,6,128,2 (2C), 135,2 (2C), 140,6; MS (EI): *m*/*z* (%): 297 (M⁺ + 1, 5), 295 (M⁺- 1, 5), 281 (3), 283 (3), 217 (100), 197 (15), 131 (13), 117 (50), 91 (12), 43 (39); IR (Film, cm⁻¹) *v*ₘₐₓ = 3044, 2974, 2919, 1937, 1609, 1512, 1396, 1356, 1320, 1269, 1217, 1143, 1085, 1017, 960, 845, 784, 655, 602.

### Darstellung von Verbindung 19

Verbindung **18** (16 g, 53,9 mmol), Phthalimid (10,3 g, 72 mmol) und K₂CO₃ (9,7 g, 72 mmol) wurden in 60 ml trockenem Aceton 12 h unter Rückfluss erhitzt. Anschließend wurde das Aceton unter vermindertem Druck abdestilliert und 100 mal Wasser zum Auflösen der anorganischen Salze hinzugegeben. Die Reaktionsmischung wurde unter vermindertem Druck filtriert, mit Wasser gewaschen und getrocknet, was Verbindung **19** (17,6 g) als weißen Feststoff ergab. Die Ausbeute betrug 90%.
¹H NMR (CDCl₃, 300 MHz): δ 1,31 (s, 12H, 4CH₃), 4,85 (s, 2H, CH₂), 7,42 (d, *J*= 6,6 Hz, 2H, ArH), 7,67-7,70 (m, 2H, ArH), 7,76 (d, *J*= 6,6 Hz, 2H, ArH), 7,81-7,84 (m, 2H, ArH); ¹³C NMR (CDCl₃, 75 MHz): δ 24,8 (4C), 41,6, 83,7 (2C), 123,3 (2C), 127,8 (2C), 132,1, 133,9 (2C), 135,1 (3C), 139,3 (2C), 167,9 (2C); MS (EI): *m*/*z* (%): 363 (M⁺, 100), 348 (17), 264 (37), 217 (34), 160 (31), 130 (29), 117 (92), 91 (16), 76 (36), 43 (78); IR (Film, cm⁻¹) *v*ₘₐₓ = 2989, 2941, 1772, 1718, 1610, 1429, 1393, 1358, 1342, 1141, 1087, 1020, 962, 938, 856, 786, 718, 659.

### Darstellung von Verbindung 21

Verbindung 19 (2,5 g, 6,9 mmol) und Hydrazinhydrat (0,47 ml, 80%, 7 mmol) wurden bei Raumtemperatur in 30 ml Methanol gelöst und die Mischung wurde auf Rückfluss erwärmt. Nach 12 h wurde auf Raumtemperatur abgekühlt und unter vermindertem Druck filtriert. Der weiße Feststoff wurde verworfen und das Filtrat zur Trockne eingeengt. Dabei wurde Wasser mittels trockenen Benzols unter Schutzgasatmosphäre entfernt. Dann wurden NEt₃ (2,9 ml), trockenes Methanol (10 ml) und Verbindung 2 (2,1 g, 14 mmol) zugegeben und die Reaktionsmischung 10 h bei Raumtemperatur gerührt. Anschließend wurden K₂CO₃ (952 mg, 7 mmol) und Verbindung 8 (1,6 g, 8,3 mmol) zugegeben und weitere 10 h bei Raumtemperatur gerührt. Danach wurde etwas Wasser zur Reaktionsmischung gegeben und mit CHCl₃ extrahiert. Es wurde mit Wasser gewaschen und die organische Phase wurde abgetrennt, getrocknet und eingeengt. Der Rückstand wurde mittels Säulenchromatographie gereinigt, was Verbindung **21** (85 mg) als Öl ergab. Die Ausbeute betrug 4%.
¹H NMR (CDCl₃, 300 MHz): δ 0,88 (t, *J*= 7,5 Hz, 3H, CH₃), 1,25-1,38 (m, 14H, CH₂ und 4CH₃), 1,63-1,73 (m, 2H, CH₂), 2,62 (t, *J*= 7,5 Hz, 2H, CH₂), 5,59 (s, 2H, ArCH₂), 6,99 (d, *J*= 7,8 Hz, 2H, ArH), 7,73 (d, *J*= 7,8 Hz, 2H, ArH), 7,78 (s, 1 H, NCH=), 9,66 (s, 1 H, CHO); ¹³C NMR (CDCl₃, 75 MHz): δ 13,7, 22,4, 24,8(4C), 26,5, 29,3, 48,2, 83,9(2C), 125,5(2C), 131,4, 135,3 (3C), 139,2, 143,6, 156,8, 178,7; MS (FAB): M⁺ = 368, gefunden: 369 (M⁺ + 1); IR (Film, cm-¹) *v*ₘₐₓ = 3044, 2975, 2933, 2870, 1671, 1614, 1533, 1464, 1405, 1362, 1326, 1270, 1160, 1145, 1089, 1021, 963, 858, 821, 793, 721, 654.

### Darstellung von Verbindung 22 und 23

Eine Suspension von o-Brombenzonitril (9,1 g, 50 mmol), NH₄Cl (3,5 g, 65 mmol), NaN₃ (4,3 g, 65 mmol) und LiCl in 80 ml DMF wurde auf 100 °C erwärmt und 12 h gerührt. Ein Großteil des Lösungsmittels wurde bei 120 °C unter vermindertem Druck abdestilliert. Der Rückstand wurde mittels einer 10%igen wässrigen Lösung von NaOH alkalisch gemacht, bis zu einem pH-Wert von 12. Die Reaktionsmischung wurde mit Ethylacetat extrahiert und die anorganische Phase mit konzentrierter HCl bis zu einem pH-Wert von 2 angesäuert, wobei ein weißer Feststoff ausschied. Dieser wurde unter vermindertem Druck mittels eines Büchner-Trichters abfiltriert, mit Wasser gewaschen und getrocknet, was Verbindung 22 (10 g, Ausbeute 90%) ergab.

Verbindung **22** wurde in 30 ml CH₂Cl₂ gelöst, die Mischung auf 0 °C im EisWasserbad abgekühlt und NEt₃ (8 ml) zugegeben. Anschließend wurde Ph₃CCl (13,2 g, 47 mmol) in 3 Portionen innerhalb von 10 min zugegeben und die Reaktionsmischung auf Raumtemperatur erwärmt. Nach 3 h rühren wurde mit einem Büchner-Trichters filtriert unter vermindertem Druck, mit Wasser gewaschen und getrocknet, was Verbindung **23** (18,9 g) ergab. Ausbeute betrug 90%.
¹H NMR (CDCl₃, 300 MHz): δ 7,18-7,36 (m ,17H, ArH), 7,66 (d, J= 7,8 Hz, 1H, ArH), 7,88 (d, J= 7,8 Hz, 1 H, ArH); ¹³C NMR (CDCl₃, 75 MHz): δ 83,3, 122,2, 127,3, 127,7, 128,3, 128,7, 130,3, 131,1, 131,6, 133,9, 141,2, 162,9.

### Darstellung von Verbindung 12

Benzonitril (10.3 g, 100 mmol), NH₄Cl (6.9 g, 1.3 eq), NaN₃ (8.5 g, 1.3 eq) und LiCl (300 mg) wurden in 100 ml DMF gelöst und die Reaktionsmischung wurde 12 h bei 100 °C gerührt. Anschließend wurde der Großteil des Lösungsmittels unter vermindertem Druck entfernt. Der Rückstand wurde mit 10%iger wässriger NaOH bis zu einem pH-Wert von pH 12 alkalisch gemacht. Nach der Extraktion mit Ethylacetat wurde die wässrige Phase abgetrennt und mit konzentrierter Salzsäure bis pH 2 angesäuert. Der Niederschlag wurde in einem Büchner-Trichter abfiltriert, mit Wasser gewaschen und getrocknet, was 5-Phenyltetrazol (13.5 g, Schmelzpunkt 208 - 209 °C) ergab. Die Ausbeute betrug 96%.
¹H NMR (d-DMSO, 300 MHz) δ 7.55-7.57 (3H, m), 8.01-8.03 (2H, m); ¹³C NMR (*d*-DMSO, 75 MHz) δ 129.5, 132.4, 134.8, 136.7, 160.7; MS (EI) *m*/*z* (%): 146 (M+, 42), 118 (100), 103 (17), 91 (46), 77 (32), 63 (48); IR (Film, cm⁻¹) *v*ₘₐₓ = 3055, 2982, 2837, 2607, 2545, 1607, 1562, 1485, 11463, 1409, 1163, 1056,1013, 725, 703, 686.

5-Phenyltetrazol (6.6 g, 45 mmol) wurde in 20 ml CH₂Cl₂ gelöst und mit NEt₃ (8 ml, 1.3 eq) versetzt. Die Reaktionsmischung wurde in einem Eiswasserbad auf 0 °C abgekühlt und Ph₃CCl (13.2 g, 1.05 eq) wurde innerhalb von 10 min in 3 Portionen zugegeben. Anschließend wurde auf Raumtemperatur erwärmt und 3 h gerührt. Die Reaktionsmischung wurde filtriert, mit Wasser gewaschen und getrocknet um Verbindung **12** (16.5 g, Schmelzpunkt 163 - 164 °C) zu erhalten. Die Ausbeute betrug 94%.
¹H NMR (CDCl₃, 300 MHz) δ 7.21-7.24 (6H, m), 7.37-7.39 (9H, m), 7.47-7.49 (3H, m), 8.19-8.20 (2H, m); ¹³C NMR (CDCl₃, 75 MHz) δ 83.0, 127.0, 127.5, 127.7, 128.3, 128.7, 130.3, 141.3, 164.0; IR (Film, cm⁻¹) *v*ₘₐₓ = 3058, 1490, 1465, 1445, 1186, 1028, 874, 763, 748, 697, 635.

### Darstellung von Verbindung 13

Eine Lösung von Verbindung **12** (10 g, 25.8 mmol) in THF (30 ml) wurde unter Argonatmosphäre auf -20 °C abgekühlt. Anschließend wurde BuLi (1 M, 27 ml, 1.05 eq) hinzugegeben. Die Temperatur wurde auf -5 °C erhöht und es wurde 1 h gerührt. In der Zwischenzeit fiel eine große Menge eines Feststoffs aus. Erneut wurde auf -25 °C abgekühlt und B(OMe)₃ (4.3 ml, 1.5 eq) über eine Spritze langsam hinzugegeben. Anschließend ließ man die Reaktionsmischung sich auf 20 °C erwärmen und rührte sie eine halbe Stunde. Das Lösungsmittel wurde unter vermindertem Druck auf 1/3 der ursprünglichen Menge reduziert wobei sich ein weißer Feststoff bildete. Der Feststoff wurde abfiltriert, gewaschen mit 20 % THF in H₂O (40 ml) und Wasser (40 ml) und getrocknet, was Verbindung 13 (10.4 g) ergab. Die Ausbeute betrug 94%. Verbindung 13 kann ohne weitere Reinigung weiterverwendet werden.

## Patentansprüche

1. Verfahren zur Herstellung einer Verbindung der allgemeinen Formel I, 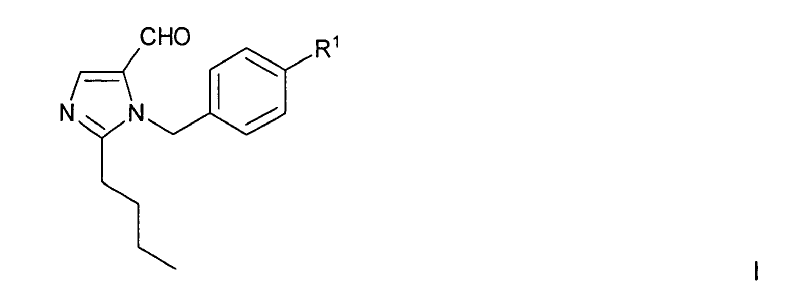 in der R¹ ein Rest R^{1a} oder ein Rest R^{1b} ist, wobei
- R^{1a} ein Rest der allgemeinen Formel II ist 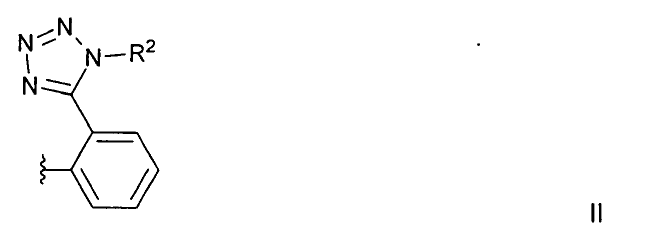 in der R² eine Tetrazol-Schutzgruppe bedeutet, oder
- R^{1b} ein Rest ist, der in der Lage ist, die Phenylengruppe der Verbindung der allgemeinen Formel I durch Reaktion mit einem hierzu komplementären Rest R³, der Bestandteil einer eine andere Phenyleneinheit enthaltenden Verbindung der allgemeinen Formel III
R³-R⁴ III
in der R⁴ ein Rest der allgemeinen Formel II ist,
unter Bildung einer C-C-Bindung zwischen der Phenylengruppe der Verbindung der allgemeinen Formel I und der Phenylengruppe der Verbindung der allgemeinen Formel III zu kuppeln,
indem man eine Verbindung der allgemeinen Formel IV 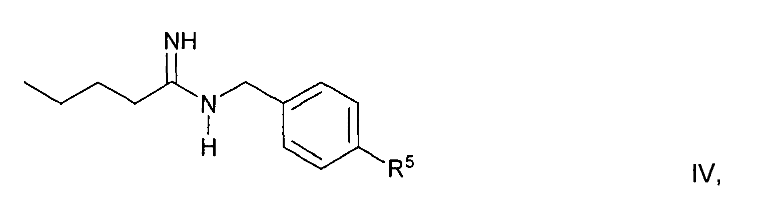 in der R⁵
- für den Fall, dass R¹ in Formel I ein Rest R^{1a} ist, ein Rest der allgemeinen Formel II bedeutet, und
- für den Fall, dass R¹ in Formel I ein Rest R^{1b} ist, die gleiche Bedeutung wie Rest R^{1b} in Formel I hat
mit einer Verbindung der der allgemeinen Formel V 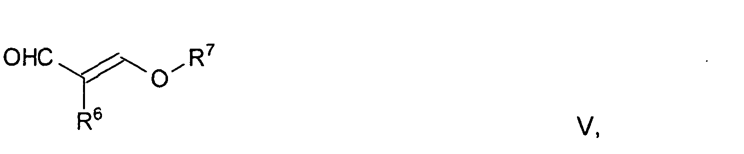 in der R⁶ ein Halogen aus der Gruppe Cl, Br, I, bevorzugt Br und R⁷ eine verzweigte oder unverzweigte C₁ - C₆-Alkylgruppe, bevorzugt eine Isopropylgruppe bedeuten, umsetzt.

2. Verfahren nach Anspruch 1, wobei die Tetrazol-Schutzgruppe R² in Formel II Triphenylmethyl oder *tet*.-Butyl bedeutet.

3. Verfahren nach einem der vorstehenden Ansprüche, wobei man die Reaktion in Gegenwart einer schwachen Bronstedt-Base durchführt.

4. Verfahren nach einem der vorstehenden Ansprüche, wobei der Rest R^{1b} der Verbindung der allgemeinen Formel I oder Rest R⁵ in der Verbindung der allgemeinen Formel IV ein Rest ist, der in der Lage ist, mit dem Rest R³ in einer Suzuki-, Stille- oder Grignard-Reaktion zu reagieren.

5. Verfahren nach einem der vorstehenden Ansprüche, wobei der Rest R^{1b} in der Verbindung der allgemeinen Formel I oder Rest R⁵ in der Verbindung der allgemeinen Formel IV die folgende Bedeutung hat:
- Halogen,
- ein Rest der allgemeinen Formel VI 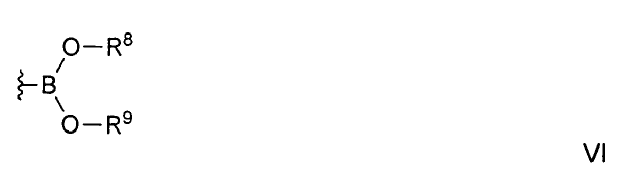 in der R⁸ und R⁹ Wasserstoff, eine C₁- bis C₆-Alkylgruppe oder gemeinsam eine C₁- bis C₆-Alkandiylgruppe bedeutet,
- ein Trialkylzinnrest oder
- wenn in dem Verfahren eine Verbindung der allgemeinen Formel I mit dem Rest R^{1b} verwendet wird, ein Magnesium(II)halogenidrest,
und wobei,
wenn R^{1b} oder R⁵ für ein Halogen steht, R³ für einen Rest der allgemeinen Formel VI, einen Trialkylzinnrest oder, wenn in dem Verfahren eine Verbindung der allgemeinen Formel I mit dem Rest R^{1b} verwendet wird, einen Magnesium(II)halogenidrest steht und umgekehrt.

6. Verbindung der allgemeinen Formel IV, in der R⁵ ein Rest der allgemeinen Formel II bedeutet.

7. Verfahren zur Herstellung der Verbindung der allgemeinen Formel IV, in der R⁵ ein Rest der allgemeinen Formel II bedeutet, in dem man eine Verbindung der allgemeinen Formel VII 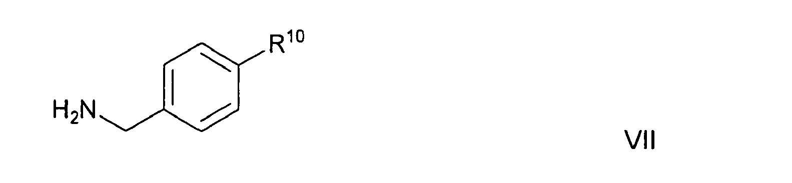 in der R¹⁰ ein Rest der Formel II bedeutet,
mit einer Verbindung der allgemeinen Formel VIII 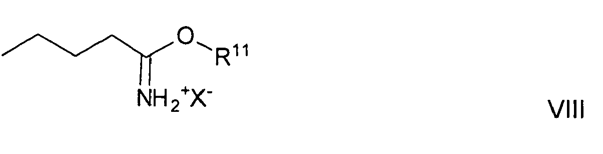 in der R¹¹ ein C₁- bis C₁₂-Alkylrest und X⁻ das Anion einer Mineralsäure bedeutet,
in Gegenwart einer Bronstedt-Base umsetzt.

8. Verfahren nach Anspruch 7, wobei man die Verbindung der Formel VII bereitstellt, indem man
I. eine Verbindung der allgemeinen Formel IX bereitstellt 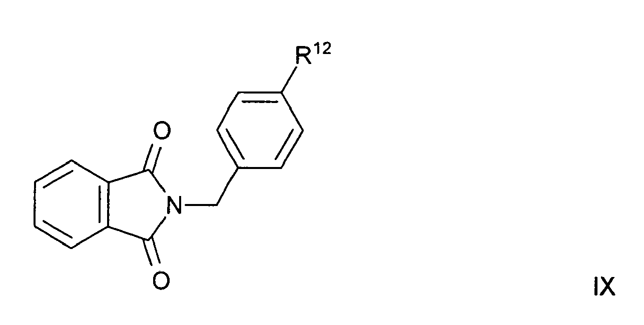 in der R¹² ein Rest der allgemeinen Formel II ist und
II. aus der Verbindung der allgemeinen Formel IX unter Bedingungen, wie sie für eine Gabriel-Reaktion üblich sind, die Verbindung der allgemeinen Formel VII herstellt.

9. Verbindung der allgemeinen Formel I, in der R¹ Halogen, insbesondere Brom, bedeutet.

10. Verbindung der allgemeinen Formel IV, in der R⁵ Halogen, insbesondere Brom, bedeutet.

11. Verfahren nach Anspruch 1 oder 2, wobei der Rest R^{1b} in Formel I bzw. der Rest R⁵ in Formel IV Brom bedeutet.

12. Verfahren zur Herstellung einer Verbindung der allgemeinen Formel IV, in der R⁵ Halogen bedeutet, wobei man ein in *para*-Stellung mit einem Halogenatom substituiertes Benzylaminderivat mit einer Verbindung der allgemeinen Formel VIII in Gegenwart einer Bronstedt-Base umsetzt.

13. Verfahren nach Anspruch 12, wobei man die Verbindung der allgemeinen Formel IV, in der R⁵ Halogen bedeutet, bereitstellt, indem man ein in *para-*Stellung mit einem Halogenatom substituiertes Benzylaminderivat in einer Gabriel-Reaktion mit Phthalimid aus einem in *para*-Stellung mit einem Halogenatom substituierten Benzylhalogenid herstellt.

14. Verfahren zur Herstellung einer Verbindung der allgemeinen Formel I mit einem Rest R^{1a}, indem man eine Verbindung der allgemeinen Formel IV, in der R⁵ Halogen bedeutet, mit einer Verbindung der allgemeinen Formel III, in der R³ ein Rest der allgemeinen Formel VI, ein Trialkylzinnrest oder ein Magnesium(II)halogenidrest bedeutet, unter Bedingungen, wie sie für eine Suzuki-, Stille- oder Grignard-Reaktion üblich sind, umsetzt.

15. Verbindung der allgemeinen Formel I, in der R¹ ein Rest der allgemeinen Formel VI, ein Trialkylzinnrest oder Magnesium(II)halogenidrest bedeutet.

16. Verbindung der allgemeinen Formel IV, in der R⁵ ein Rest der allgemeinen Formel VI, ein Trialkylzinnrest oder Magnesium(II)halogenidrest bedeutet.

17. Verfahren nach Anspruch 5, in der R⁸ und R⁹ in Formel VI gemeinsam 2,3-Dimethylbutan-2,3-diyl bedeuten.

18. Verfahren zur Herstellung einer Verbindung der allgemeinen Formel IV, in der R⁵ ein Rest der allgemeinen Formel VI bedeutet, in dem man ein in *para*-Stellung mit einem Rest R⁵ der allgemeinen Formel VI substituiertes Benzylaminderivat mit einer Verbindung der allgemeinen Formel VIII in Gegenwart einer Bronstedt-Base umsetzt.

19. Verfahren nach Anspruch 18, wobei man eine Verbindung der allgemeinen Formel IV, in der R⁵ ein Rest der allgemeinen Formel VI bedeutet, bereitstellt, indem man ein in *para-*Stellung mit einem Rest R⁵ der allgemeinen Formel VI substituiertes Benzylaminderivat in einer Gabriel-Reaktion mit Phthalimid aus einem in *para*-Stellung mit einem Rest R⁵ der allgemeinen Formel VI substituiertes Benzylhalogenid, herstellt.

20. Verfahren zur Herstellung einer Verbindung der allgemeinen Formel I, in der R¹ ein Rest der allgemeinen Formel II bedeutet, indem man eine Verbindung der allgemeinen Formel IV, in der R⁵ ein Rest der allgemeinen Formel VI bedeutet, mit einer Verbindung der allgemeinen Formel III, in der R³ Halogen bedeutet, unter Bedingungen, wie sie für eine Suzuki-Reaktion üblich sind, umsetzt.

21. Verbindung der allgemeinen Formel XI, 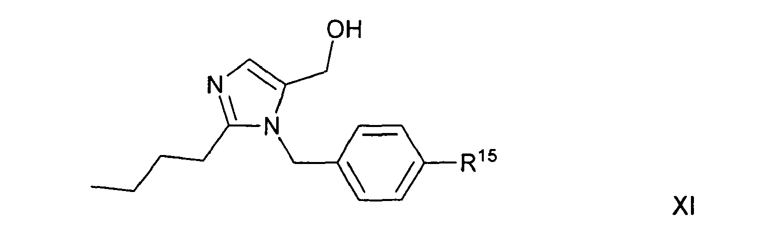 in der R¹⁵ ein Rest der allgemeinen Formel II bedeutet.

22. Verfahren zur Herstellung von Losartan oder eines seiner pharmakologisch akzeptablen Salze nach Anspruch 1 oder 2, in dem man
a) in einem Schritt (a) ausgehend von einer Verbindung der allgemeinen Formel I mit einem Rest R^{1a} die Verbindung der allgemeinen Formel XI herstellt, indem man die Formylgruppe, mit der die Imidazolgruppe substituiert ist, auf übliche Weise zu einer Hydroxymethylgruppe reduziert,
b) in einem Schritt (b) das einzige noch verbliebene Wasserstoffatom in der Imidazolgruppe der nach Schritt (a) hergestellten Verbindung auf übliche Weise durch Chlor ersetzt und
(c) in einem Schritt (c) die Tetrazol-Schutzgruppe abspaltet und gegebenenfalls
(d) aus Losartan eines seiner pharmakologisch akzeptablen Salze herstellt.

23. Verfahren nach einem oder mehreren der Ansprüche 1 bis 5, 11, 14, 17, 20 und 22, in dem ein oder mehrere Katalysatoren umfassend ein oder mehrere Übergangsmetalle, vorzugsweise ausgewählt aus MnCl₂, CrCl₃, FeCl₂, Fe(acac)₃, FeCl₃, Fe(salen)Cl, NiCl₂(PPh₃)₂, CoCl₂(dppe), CoCl₂(dpph), Co(acac)₂, CoCl₂(dppb), PdCl₂(PPh₃)₂ oder Pd(PPh₃)₄, zum Einsatz kommen.

24. Verfahren zur Herstellung von einem Imidazolderivat, das an einem oder mehreren Kohlenstoffatomen des Imidazolrings mit Chlor substituiert ist (Imidazolderivat A), in dem man Imidazol oder ein Imidazolderivat, das an mindestens einem Kohlenstoffatom des Imidazolrings ein Wasserstoffatom trägt (Imidazolderivat B), mit CeCl₃ und einem Alkalisalz einer Hypohalogensäure umsetzt.

25. Verfahren nach Anspruch 24, wobei man als Imidazolderivat (A) eine Verbindung herstellt, die an dem Kohlenstoffatom des Imidazolrings in der 4- oder 5-Position oder an beiden vorgenannten Positionen mit Chlor substituiert ist, und wobei man als Imidazolderivat (B) eine Verbindung einsetzt, die an dem Kohlenstoffatom des Imidazolrings in der 4- oder 5-Position oder an beiden vorgenannten Positionen noch ein Wasserstoffatom trägt.

26. Verfahren nach Anspruch 24 oder 25, wobei man ein Losartan-Derivat herstellt, bei dem das Wasserstoffatom der Tetrazolgruppe durch eine Tetrazol-Schutzgruppe ersetzt ist, und wobei man als Imidazolderivat (B) die Verbindung der allgemeinen Formel XI einsetzt.

27. Verfahren nach einem oder mehreren der Ansprüche 24 bis 26, wobei man das CeCl₃ und das Alkalisalz einer Hypohalogensäure in stöchiometrischen Mengen oder im Überschuss einsetzt.

28. Verfahren nach einem oder mehreren der Ansprüche 24 bis 27, wobei man als Alkalisalz der Hypohalogensäure ein Kalium- oder Natriumsalz einsetzt.

29. Verfahren nach einem oder mehreren der Ansprüche 24 bis 28, wobei man als Alkalisalz der Hypohalogensäure ein Alkalisalz der hypochlorigen Säure einsetzt.

30. Verfahren nach einem oder mehreren der Ansprüche 24 bis 29, wobei man die Reaktion in einem 2-phasigen System durchführt, bei dem eine Phase aus einer wässrigen Lösung gebildet ist und die andere Phase aus einer Lösung gebildet wird, die ein mit Wasser nicht unbegrenzt mischbares organisches Lösungsmittel umfasst.

31. Verfahren nach Anspruch 22, wobei man in Schritt (b) das einzige noch verbliebene Wasserstoffatom in der Imidazolgruppe der nach Schritt (a) hergestellten Verbindung auf übliche Weise durch Chlor ersetzt, indem man die nach Schritt (a) hergestellte Verbindung mit CeCl₃ und einem Alkalisalz einer Hypohalogensäure als Reagenzien umsetzt.
